(19) Europäisches Patentamt / European Patent Office / Office européen des brevets

(11) **EP 4 212 551 A1**

(12) **EUROPEAN PATENT APPLICATION**
published in accordance with Art. 153(4) EPC

(43) Date of publication:
**19.07.2023 Bulletin 2023/29**

(21) Application number: **21867165.9**

(22) Date of filing: **10.09.2021**

(51) International Patent Classification (IPC):
*C07K 16/28* (2006.01)   *A61P 37/08* (2006.01)
*A61P 29/00* (2006.01)   *G01N 33/68* (2006.01)
*A61K 39/00* (2006.01)

(52) Cooperative Patent Classification (CPC):
**A61K 39/00; A61P 29/00; A61P 37/08;
C07K 16/28; G01N 33/68**

(86) International application number:
**PCT/KR2021/012345**

(87) International publication number:
**WO 2022/055300 (17.03.2022 Gazette 2022/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.09.2020 KR 20200117668**

(71) Applicant: **Ajou University Industry-Academic
Cooperation
Foundation
Gyeonggi-do 16499 (KR)**

(72) Inventors:
• **KIM, Yong Sung**
  **Suwon-si Gyeonggi-do 16504 (KR)**
• **PARK, Hae-Sim**
  **Seoul 06718 (KR)**
• **KIM, Jung Eun**
  **Seoul 01015 (KR)**
• **JENG, Keunok**
  **Suwon-si Gyeonggi-do 16501 (KR)**
• **LEE, Dong-Hyun**
  **Suwon-si Gyeonggi-do 16532 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **ANTIBODY BINDING HUMAN IL-5R? AND USE THEREOF**

(57)   The present invention relates to: a humanized antibody or antigen-binding fragment thereof that binds to the human IL-5 receptor alpha subunit (IL-5Rα), which is a receptor of human interleukin-5 (IL-5); a nucleic acid encoding same; a vector comprising the nucleic acid; a cell transformed with the vector; a method for producing the antibody or antigen-binding fragment thereof; a conjugate comprising the same; a bispecific or multispecific antibody comprising same; a composition comprising same, for preventing or treating an allergic disease, an inflammatory disease and/or a disease caused by an increase in eosinophils; and a composition for diagnosis of allergic diseases, inflammatory diseases, and/or diseases caused by an increase in eosinophils.

【Fig. 13a】

EP 4 212 551 A1

## Description

[Technical Field]

The present invention relates to a humanized antibody or antigen-binding fragment thereof that binds to human IL-5 receptor alpha subunit (IL-5Ra), which is a receptor for human interleukin-5 (IL-5), a nucleic acid encoding the same, a vector including the nucleic acid, cells transformed with the vector, a method for producing the antibody or antigen-binding fragment thereof, a conjugate including the same, a bispecific or multispecific antibody including the same, a composition for preventing or treating allergic diseases, inflammatory diseases, and/or diseases caused by an increase in eosinophils containing the same, and a composition for diagnosing the allergic diseases, inflammatory diseases, and/or diseases caused by an increase in eosinophils containing the same.

[Background Art]

Human interleukin 5 (hereinafter referred to as "IL-5") is a type of cytokine secreted by T-cells or mast cells. IL-5 in mice is known to act as a differentiation and proliferation factor for B-cells and eosinophils. It is known that the IL-receptor acts mainly as a differentiation and growth factor of eosinophils in the human body. It has become clear that the IL-5 receptor is composed of an $\alpha$ chain (hereinafter referred to as IL-5R$\alpha$) and a $\beta$ chain (hereinafter referred to as IL-5R$\beta$ or $\beta$c receptor) . In addition, IL-5R$\alpha$ is involved in direct IL-5 binding, and the $\beta$c receptor itself does not exhibit IL-5 binding ability, but is responsible for intracellular signaling. In addition, the $\beta$c receptor is known to act as a common receptor with receptors such as interleukin-3 (hereinafter referred to as IL-3) and a granulocyte-macrophage colony-stimulating factor (hereinafter referred to as GM-CSF).

The IL-5 receptor is known to be expressed only in eosinophils and basophils and in some mast cells in the human body. IL-5 is essential for eosinophil maturation, proliferation and activation and can enhance basophil differentiation. IL-5 promotes tissue accumulation of eosinophils through the ability thereof to upregulate the expression of vestibular adhesion molecules upon migration of eosinophils to other tissues. IL-5 also prolongs eosinophil survival by blocking eosinophil apoptosis. The main cellular sources of IL-5 are Th2 cells (Type 2 helper T cells), ILC2 (type 2 innate lymphoid cells), mast cells and natural killer T cells.

Eosinophils are circulating granulocytes produced in the bone marrow and are a type of major cells recruited to sites of inflammatory responses. It is known that the number of eosinophils in the blood of patients in allergic diseases such as chronic bronchial asthma is increased. Eosinophils function to release toxic granule proteins, reactive oxygen species (ROS) cytokines, and lipid mediators. Therefore, an increase in eosinophils is known to be involved in the development of various inflammatory diseases including allergic diseases related to hypersensitivity reactions in lung tissue (Possa et al., 2013). The close association between eosinophils and IL-5 leads to the development of novel drugs that are capable of inhibiting the activity of eosinophils by directly acting on IL-5 and IL-5R$\alpha$ and treating allergic diseases such as chronic bronchial asthma.

Mepolizumab is an N-glycosylated humanized IgG1 antibody that binds to IL-5 and inhibits the binding to IL-5R$\alpha$. Mepolizumab has been tested in several diseases including severe asthma, atopic dermatitis and nasal polyposis. Mepolizumab was approved by the FDA in 2015 based on excellent results in asthma patients. The amount of eosinophils decreased in subjects treated with mepolizumab, but serum IL-5 levels were found to increase over time (Pouliquen et al., 2015; Tsukamoto et al., 2016). One group hypothesizes that most IL-5 detected during treatment is part of a complex bound to immunoglobulins (mostly mepolizumab) and prolongs the half-life of complexed IL-5. The biological significance and fate of this complex are unknown.

Reslizumab is a humanized IgG4 antibody that binds to IL-5 with high affinity to block the biological functions thereof. It was approved for use as an add-on maintenance therapy in patients with severe eosinophilic asthma in the United States and Europe. Like mepolizumab, reslizumab increased serum IL-5 levels after 1-month treatment of patients with hypereosinophilic syndrome (HES). However, it is still unknown whether or not this is due to free or complex IL-5. Culture medium-containing serum from reslizumab-treated patients has been shown to prolong eosinophil survival *in vitro,* and researchers have hypothesized that anti-IL-5 not only prolongs half-life, but actually improves IL-5 activity under certain conditions (Roufosse, 2018).

Benralizumab is an afucosylated humanized IgG1 antibody and has two mechanisms different from anti-IL-5 antibodies because it targets IL-5R$\alpha$. Benralizumab 1) inhibits cell growth by blocking the action of IL-5, and 2) induces antibody-dependent cellular cytotoxicity (ADCC) by natural killer cells and macrophages. In addition, the afucosylated Fc improves the affinity for Fc$\gamma$RIIIa expressed on the surface of natural killer (NK) cells and macrophages, resulting in a stronger ADCC effect. This dual mechanism of action of benralizumab results in a much faster and higher level of depletion of eosinophils than that induced by other monoclonal antibodies targeting the IL-5 pathway, such as mepolizumab and reslizumab (Davila Gonzalez et al., 2019). Therefore, benralizumab is proposed as a therapeutic agent for not only eosinophilic asthma but also chronic obstructive pulmonary disease (COPD), eosinophilic syndrome, and chronic rhinitis.

According to research reported to date, targeting IL-5Rα rather than IL-5 has the advantage of directly lowering the number of eosinophils without increasing the half-life of IL-5. However, no therapeutic anti-IL-5Rα antibody other than benralizumab has yet been approved, and benralizumab did not show significant effects in some patients. In addition, the continuous and repeated administration of one therapeutic agent may cause resistance to the therapeutic agent, such as the generation of anti-drug antibodies (ADA). Accordingly, the technical demand for an anti-IL-5Rα antibody having a strong therapeutic effect is still high.

Under this technical background, the inventors of the present application tried to develop a humanized antibody specific to IL-5Rα that has different epitopes and higher affinity and thus exhibits excellent efficacy, although it is an antibody to the same antigen. In the present invention, 1) an antibody that inhibits the activity of IL-5 with higher affinity than conventional benralizumab antibodies was developed. In addition, unlike benralizumab, which has an epitope in the membrane-distal domain, an anti-IL-5Rα humanized antibody having higher antibody-dependent cell cytotoxicity (ADCC) by targeting the epitope in the membrane-proximal domain was developed. Based thereon, the present invention was completed.

[Disclosure]

[Technical Problem]

Therefore, the present invention has been made in view of the above problems, and it is one object of the present invention to provide a humanized antibody or antigen-binding fragment thereof having high affinity and specificity for IL-5Rα.

It is another object of the present invention to provide a humanized antibody or antigen-binding fragment thereof that has increased antagonistic activity due to the low antigen dissociation constant to IL-5Rα.

It is another object of the present invention to provide a humanized antibody or antigen-binding fragment thereof that has a membrane-proximal domain as an epitope for an antigen of IL-5Rα.

It is another object of the present invention to provide a humanized antibody or antigen-binding fragment thereof that inhibits IL-5-dependent cell proliferation of eosinophils and basophils that target and express IL-5Rα.

It is another object of the present invention to provide a humanized antibody or antigen-binding fragment thereof that induces antibody-dependent cellular cytotoxicity (ADCC) by natural killer (NK) cells and macrophages by binding to eosinophils and basophils that target and express IL-5Rα.

It is another object of the present invention to provide a humanized antibody or antigen-binding fragment thereof that binds to IL-5Rα with high affinity, has a membrane-proximal domain as an epitope, and thus strongly induces antibody-dependent cellular cytotoxicity (ADCC) by natural killer (NK) cells and macrophages for eosinophils and basophils that express IL-5Rα.

It is another object of the present invention to provide nucleic acid encoding the antibody or antigen-binding fragment thereof.

It is another object of the present invention to provide a vector containing the nucleic acid, a cell transformed with the vector, and a method for producing the same.

It is another object of the present invention to provide a conjugate including the antibody or antigen-binding fragment thereof.

It is another object of the present invention to provide a pharmaceutical composition for preventing or treating allergic diseases, inflammatory diseases, and/or diseases caused by an increase in eosinophils, containing the antibody or antigen-binding fragment thereof.

It is another object of the present invention to provide a composition for diagnosing allergic diseases, inflammatory diseases, and/or diseases caused by an increase in eosinophils, containing the antibody or antigen-binding fragment thereof.

[Technical Solution]

In accordance with one aspect of the present invention, the above and other objects can be accomplished by the provision of an antibody or antigen-binding fragment thereof binding to human IL-5Rα recognizing, as an epitope, at least one amino acid residue selected from the group consisting of amino acid residues 221 to 322 corresponding to domain 3 (D3) of a sequence of human IL-5 receptor alpha subunit (IL-5Rα) represented by SEQ ID NO: 19.

In accordance with another aspect of the present invention, provided is an antibody or antigen-binding fragment thereof binding to human IL-5Rα including a heavy-chain CDR1 of SEQ ID NO: 3, a heavy-chain CDR2 selected from the group consisting of SEQ ID NOS: 4, 15 to 18, a heavy-chain CDR3 selected from the group consisting of SEQ ID NOS: 5, 27 to 32, and a light-chain CDR1 of SEQ ID NO: 8, a light-chain CDR2 of SEQ ID NO: 9, and a light-chain CDR3 of SEQ ID NO: 10.

In accordance with another aspect of the present invention, provided are a nucleic acid encoding the antibody or antigen-binding fragment thereof and an expression vector containing the nucleic acid.

In accordance with another aspect of the present invention, provided is a cell transformed with the expression vector.

In accordance with another aspect of the present invention, provided is a method of producing an antibody or antigen-binding fragment thereof binding to human IL-5Rα.

In accordance with another aspect of the present invention, provided is a composition including T cells expressing the antibody or antigen-binding fragment thereof as a chimeric antigen receptor.

In accordance with another aspect of the present invention, provided is a conjugate including the antibody or antigen-binding fragment thereof and a drug.

In accordance with another aspect of the present invention, provided is a bispecific or multispecific antibody including the antibody or antigen-binding fragment thereof.

In accordance with another aspect of the present invention, provided is a composition for preventing or treating allergic diseases, inflammatory diseases, and/or diseases caused by an increase in eosinophils including the antibody or antigen-binding fragment thereof.

In accordance with another aspect of the present invention, provided is a composition for diagnosing allergic diseases, inflammatory diseases, and/or diseases caused by an increase in eosinophils including the bispecific antibody or antigen-binding fragment thereof.

[Description of Drawings]

FIG. 1 is a schematic diagram illustrating a recombinant expression vector to express an antigen protein in the sequence of human IL-5 receptor alpha subunit (referred to as "sIL-5Rα") represented by SEQ ID NO: 19, which is an extracellular domain of IL-5Rα, wherein Pcmv represents a promoter, A represents an Avi tag, and H represents a poly 6 × histidine (6×His) Tag.

FIG. 1B shows the results of SDS-PAGE to identify the sIL-5Rα antigen protein expressed in HEK293F cells, wherein NR represents a non-reducing condition, R represents a reducing condition, and M represents a protein marker.

FIG. 2A shows the result of indirect ELISA to identify binding affinity of mouse antibodies to sIL-5Rα obtained through mouse immunization to the sIL-5Rα antigen protein.

FIG. 2B shows the result of evaluation of inhibitory activity against IL-5-dependent cell proliferation of mouse antibody to IL-5Rα and benralizumab analogue using TF-1/IL-5Rα cells, which are a cell line obtained by overexpressing IL-5Rα in TF-1 cells.

FIG. 3A shows binding isotherms obtained by analyzing the affinity of the mouse antibody m2B7 and the hu2B7 antibody constructed by humanizing m2B7.

FIG. 3B shows the result of comparison in the IL-5-dependent cell growth inhibition ability in TF-1/IL-5Rα cells between the humanized antibody hu2B7, the mouse antibody mB7 and the benralizumab analogue.

FIG. 4 is a schematic diagram illustrating a strategy for constructing a library based on hu2B7 to improve the affinity of an antibody to sIL-5Rα, wherein the library was constructed in the CDR2 domain of the heavy-chain variable region using the NHB degenerate codon.

FIG. 5A shows binding isotherms obtained by analyzing the affinity of the selected anti-IL-5Rα humanized antibodies.

FIG. 5B shows the results of indirect ELISA to identify the specific binding of the selected anti-IL-5Rα humanized antibodies to the sIL-5Rα antigen protein.

FIG. 5C shows the result of SDS-PAGE purified IL-5-mFc ligand.

FIG. 5D shows the result of binding competition ELISA of selected anti-IL-5Rα humanized antibodies and IL-5-mFc ligand to sIL-5Rα, wherein binding competition with IL-5-mFc was determined depending on the concentration of the antibodies, which indicates that the anti-IL-5R$\alpha$ antibodies bind to sites overlapping with the IL-5 binding site.

FIG. 6A shows the results of evaluation of the ability of selected anti-IL-5R$\alpha$ humanized antibodies to inhibit IL-5-dependent cell growth in TF-1/IL-5R$\alpha$ cells compared with a benralizumab analogue.

FIG. 6B shows the result of comparison in the IL-5-dependent cell proliferation inhibitory ability between anti-IL-5R$\alpha$ humanized antibodies (5R65 and 5R86) in eosinophils from patients with severe eosinophilic asthma (SEA) and the benralizumab analogues.

FIG. 7A shows a structural composite of the extracellular domain of IL-5R$\alpha$ and IL-5 (PDB ID: 3QT2).

FIG. 7B is a schematic diagram illustrating three types of human IL-5 receptors (IL-5R$\alpha$), mouse IL-5 receptors (mIL-5R$\alpha$), and modified receptors (IL-5R$\alpha$ variants) of human/mouse IL-5 receptors mixed with extracellular domains expressed on the surface of yeast.

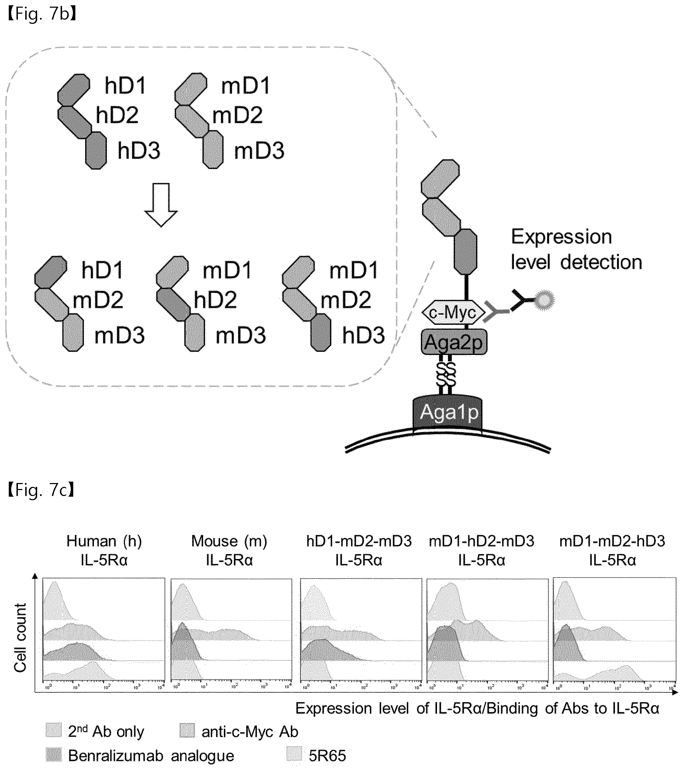

FIG. 7C shows the result of flow cytometry to determine the domain of human IL-5R$\alpha$ to which the benralizumab analogue and the 5R65 antibody bind, wherein the two antibodies bound to human IL-5R$\alpha$, but did not bind to mouse IL-5R$\alpha$, and the result of evaluation of the binding ability to human-mouse modified receptors showed that benralizumab analogues have binding ability to hIL-5R$\alpha$ or hD1-mD2-mD3 IL-5R$\alpha$ that include amino acid residues Asp1 to Gly103 corresponding to domain 1 (D1) of the sequence of human IL-5R$\alpha$ represented by SEQ ID NO: 19 and 5R65 exhibits binding ability to hIL-5R$\alpha$ or mD1-mD2-hD3 IL-5R$\alpha$ that include amino acid residues Pro221 to Trp322 corresponding to domain 3 (D3) of the sequence of human IL-5R$\alpha$ represented by SEQ ID NO: 19.

FIG. 8A is a schematic diagram illustrating a strategy for constructing a library based on 5R65 to improve the affinity of an antibody to sIL-5R$\alpha$, wherein the library was constructed using spiked oligonucleotides that are capable of retaining the original amino acid of 5R65 at each residue with a probability of 50% in CDR3 of the heavy-chain variable region and CDR3 of the light-chain variable region.

FIG. 8B shows binding isotherms obtained by analyzing the affinity of the selected anti-IL-5R$\alpha$ humanized antibodies.

FIG. 8C shows domain mapping to identify that the anti-IL-5R$\alpha$ humanized antibody has an epitope different from the benralizumab analogue.

FIG. 8D shows the results of evaluation of the inhibitory activity on IL-5-dependent cell proliferation of selected anti-IL-5R$\alpha$ humanized antibodies at various antibody concentrations in TF-1/IL-5R$\alpha$ cells compared to a benralizumab analogue.

FIG. 9A shows the result of flow cytometry to analyze the expression of IL-5R$\alpha$ in eosinophils (Siglec-8 expressing cells) in the granulocyte layers (including eosinophils and neutrophils) isolated from peripheral blood of healthy controls.

FIG. 9B shows the result of flow cytometry to analyze the expression of IL-5R$\alpha$ in eosinophils (Siglec-8 non-expressing cells) in the granulocyte layers (including eosinophils and neutrophils) isolated from peripheral blood of healthy controls.

FIG. 10A shows the result of flow cytometry to analyze the expression of IL-5R$\alpha$ in eosinophils (Siglec-8 expressing cells) in the granulocyte layers (including eosinophils and neutrophils) isolated from peripheral blood of patients with severe eosinophilic asthma (SEA).

FIG. 10B shows the result of flow cytometry to analyze the expression of IL-5R$\alpha$ in eosinophils (Siglec-8 non-expressing cells) in the granulocyte layers (including eosinophils and neutrophils) isolated from peripheral blood of patients with severe eosinophilic asthma (SEA).

FIG. 11A shows the ratio between neutrophils and eosinophils in the granulocyte layers (including eosinophils and neutrophils) isolated from peripheral blood of healthy donors and patients with severe asthma.

FIG. 11B shows the IL-5R$\alpha$ expression ratio between neutrophils and eosinophils in the granulocyte layers (including eosinophils and neutrophils) isolated from peripheral blood of healthy donors and patients with severe asthma.

FIG. 11C shows the expression levels of IL-5Rα in eosinophils in the granulocyte layers (including eosinophils and neutrophils) isolated from peripheral blood of healthy donors and patients with severe asthma.

FIG. 12A shows the result of evaluation of eosinophil proliferation inhibition ability of the anti-IL-5Rα antibody using eosinophils purified from peripheral blood of healthy donors.

FIG. 12B shows the result of evaluation of eosinophil proliferation inhibition ability of the anti-IL-5Rα antibody using eosinophils purified from peripheral blood of patients with severe asthma.

FIG. 13A is a schematic diagram illustrating the antibody-dependent cellular cytotoxicity (ADCC) predicted depending on the epitope of benralizumab analogue and 5R65.7.

FIG. 13B shows the results of evaluation of antibody-dependent cellular cytotoxicity (ADCC)-inducing ability of anti-IL-5Rα humanized antibodies using eosinophils and natural killer cells (NK cells) purified from peripheral blood of healthy donors.

FIG. 13C shows the results of evaluation of antibody-dependent cellular cytotoxicity (ADCC)-inducing ability of anti-IL-5Rα humanized antibodies using eosinophils and NK cells purified from peripheral blood of patients with severe asthma.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

According to the present invention, a novel anti-IL-5Rα humanized antibody was developed by performing mouse immunization and humanization.

In an embodiment of the present invention, a TF0-1/IL-5Rα cell line stably expressing IL-5Rα was constructed using a lentiviral system expressing the $\beta$c receptor and the biological activity of the humanized anti-IL-5Rα antibody was evaluated.

The present invention provides a method for improving the ability to inhibit the biological activity of IL-5 through affinity maturation of antibodies using a yeast expression system and four types of anti-IL-5Ra humanized antibodies.

In another embodiment of the present invention, a method for constructing yeasts expressing human IL-5Rα, mouse IL-5Rα, and human-mouse IL-5Rα receptor variants using a yeast cell surface display system is provided to identify the binding site of the antibody and thus it was found that the anti-IL-5Rα humanized antibody provided according to the present invention has a different epitope from previously developed antibodies.

In order to improve the antagonistic activity of the anti-IL-5Rα humanized antibody, affinity maturation was performed again using the yeast cell expression system described above, and kinetic screening was performed to select antibodies with a slow dissociation rate. As a result, six anti-IL-5Rα humanized antibodies were provided.

The IL-5 biological activity inhibition and antibody-dependent cellular cytotoxicity of the antibody with increased affinity were evaluated using the TF-1/IL-5Rα cell line described above and eosinophils derived from healthy donors and asthmatic patients.

Based thereon, the present inventors provide a novel anti-IL-5Rα humanized antibody or antigen-binding fragment thereof having high affinity, and increased IL-5 biological activity inhibition and antibody-dependent cytotoxic activity.

Therefore, the present invention is directed to an antibody or antigen-binding fragment thereof binding to human IL-5Rα recognizing, as an epitope, at least one amino acid residue selected from the group consisting of amino acid residues 221 to 322 corresponding to domain 3 (D3) of a sequence of human IL-5 receptor alpha subunit (IL-5Rα) represented by SEQ ID NO: 19.

In an embodiment of the present invention, the antibody competitively binds to human interleukin-5 (IL-5) and IL-5Rα, and recognizes, as an epitope, at least one amino acid residue selected from the group consisting of amino acid residues 221 to 322 corresponding to domain 3 (D3) of the sequence of human IL-5 receptor alpha subunit (IL-5Rα) represented by SEQ ID NO: 19.

As used herein, the term "epitope" refers to a protein determinant to which an antibody can specifically bind. Epitopes usually consist of a group of chemically active surface molecules, such as amino acid or sugar side chains, and generally have not only specific three-dimensional structural characteristics but also specific charge characteristics. Three-dimensional epitopes are distinguished from non-three-dimensional epitopes in that a bond to the former is broken in the presence of a denatured solvent, while a bond to the latter is not broken.

The antibody described herein functions to mediate killing of cells expressing IL-5Rα owing to higher affinity for IL-5Rα compared to benralizumab analogues and enhanced antibody-dependent cellular cytotoxicity (ADCC).

The antibody according to the present invention provides compositions and methods for treating diseases associated with IL-5 activity, such as allergic diseases. The composition contains an antibody recognizing an epitope on the extracellular domain of the human IL-5 receptor (IL-5Ra), or an antigen-binding fragment thereof. The antibody has the ability to inhibit the biological activity of IL-5 and induces the death of cells (eosinophils, etc.) expressing IL-5Rα. Therefore, the present invention can be used for diagnosis and treatment of allergic diseases such as chronic bronchial asthma and atopic dermatitis.

The present invention is directed to an antibody or antigen-binding fragment thereof binding to human IL-5Rα including a heavy-chain CDR1 of SEQ ID NO: 3, a heavy-chain CDR2 selected from the group consisting of SEQ ID NOS: 4, 15 to 18, a heavy-chain CDR3 selected from the group consisting of SEQ ID NOS: 5, 27 to 32, and a light-chain CDR1 of SEQ ID NO: 8, a light-chain CDR2 of SEQ ID NO: 9, and a light-chain CDR3 of SEQ ID NO: 10.

As used herein, the term "antibody" refers to an anti-IL-5Rα antibody that specifically binds to IL-5Rα. A complete antibody and antigen-binding fragment of the antibody molecules falls within the scope of the present invention.

The whole antibody has a structure having two full-length light-chains and two full-length heavy-chains, and each light-chain is bonded to the heavy-chain by a disulfide bond.

As used herein, the term "heavy-chain" encompasses both a full-length heavy-chain, which includes a variable domain (VH) containing an amino acid sequence having a sufficient variable region sequence for imparting specificity to an antigen and three constant domains (CH1, CH2 and CH3), and a fragment thereof. As used herein, the term "light-chain" encompasses both a full-length light-chain, which includes a variable domain (VL) containing an amino acid sequence having a sufficient variable region sequence for imparting specificity to an antigen and a constant domain (CL), and a fragment thereof.

The whole antibody includes subtypes of IgA, IgD, IgE, IgM and IgG, and in particular, IgG includes IgG1, IgG2, IgG3 and IgG4. The heavy-chain constant region has gamma ($\gamma$), mu ($\mu$), alpha ($\alpha$), delta ($\delta$) and epsilon ($\varepsilon$) types, and is subclassified into gamma 1 ($\gamma1$), gamma 2 ($\gamma2$), gamma 3 ($\gamma3$), gamma 4 (y4), alpha 1 ($\alpha1$), and alpha 2 ($\alpha2$). The light-chain constant region has kappa ($\kappa$) and lambda ($\lambda$) types.

The antigen-binding fragment of an antibody or antibody fragment refers to a fragment that has antigen-binding function and includes Fab, F(ab'), F(ab')2, Fv and the like. Among the antibody fragments, Fab refers to a structure including a variable region of each of the heavy-chain and the light-chain, the constant region of the light-chain, and the first constant domain (CH1) of the heavy-chain, each having one antigen-binding site. Fab' is different from Fab in that it further includes a hinge region including at least one cysteine residue at the C-terminus of the CH1 domain of the heavy-chain. F(ab')2 is created by a disulfide bond between cysteine residues in the hinge region of Fab'.

Fv is the minimal antibody fragment having only a heavy-chain variable region and a light-chain variable region. Two-chain Fv is a fragment in which the variable region of the heavy-chain and the variable region of the light-chain are linked by a non-covalent bond, and single-chain Fv (scFv) is a fragment in which the variable region of the heavy-chain and the variable region of the light-chain are generally linked by a covalent bond via a peptide linker therebetween, or are directly linked at the C-terminal, forming a dimer-shaped structure, like the two-chain Fv. Such antibody fragments may be obtained using proteases (e.g., Fab can be obtained by restriction-cleaving the complete antibody with papain, and the F(ab')2 fragment may be obtained by restriction-cleaving the complete antibody with pepsin), and may be produced using genetic recombination techniques.

An "Fv" fragment is an antibody fragment containing complete antibody recognition and binding sites. Such a region includes a dimer that consists of one heavy-chain variable domain.

A "Fab" fragment contains a variable domain and a constant domain of the light-chain and a variable domain and a first constant domain (CH1) of the heavy-chain. A F(ab')$_2$ antibody fragment generally includes a pair of Fab fragments covalently linked near the carboxyl terminal thereof via a hinge cysteine therebetween.

The "single chain Fv" or "scFv" antibody fragment includes VH and VL domains of the antibody, wherein these domains are present in a single polypeptide chain. The Fv polypeptide may further include a polypeptide linker between the VH domain and the VL domain in order for the scFv to form a target structure for antigen binding.

In an embodiment, the antibody according to the present invention includes, but is not limited to, monoclonal antibodies, multispecific antibodies, human antibodies, humanized antibodies, chimeric antibodies, scFVs, Fab fragments, F(ab') fragments, disulfide-bond Fvs (sdFVs), anti-idiotypic (anti-Id) antibodies, epitope-binding fragments of such antibodies, and the like.

The heavy-chain constant region may be selected from gamma ($\gamma$), mu (u), alpha ($\alpha$), delta ($\delta$) and epsilon (c) isotypes. For example, the constant region may be gamma 1 (IgG1), gamma 3 (IgG3), or gamma 4 (IgG4). The light-chain constant region may be kappa or lambda.

The term "monoclonal antibody" refers to an identical antibody, which is obtained from a population of substantially homogeneous antibodies, that is, each antibody constituting the population, excluding possible naturally occurring mutations that may be present in trivial amounts. Monoclonal antibodies are highly specific and are thus induced against a single antigenic site. Unlike conventional (polyclonal) antibody preparations that typically include different antibodies directed against different determinants (epitopes), each monoclonal antibody is directed against a single determinant on the antigen.

For example, monoclonal antibodies useful in the present invention may be produced by hybridoma methods, or may be produced in bacterial, eukaryotic or plant cells using recombinant DNA methods. In addition, monoclonal antibodies may be isolated from phage antibody libraries.

The term "affinity" refers to the ability to specifically recognize a specific site of an antigen and to bind thereto, and specificity and high-affinity of an antibody for an antigen are important factors in the immune response. The affinity constant ($K_D$) can be determined using surface plasmon resonance (SPR), for example, a BIAcore system. An affinity constant ($K_D$) calculated from a 1:1 Langmuir binding model (simultaneously $k_{on}$ and $k_{off}$) and the ratio of the rate constant $k_{off}/k_{on}$ was obtained based on surface plasmon resonance data.

The binding affinity of the anti-IL-5Rα antibody to IL-5Rα ranges from $10^{-5}$M to $10^{-12}$ M. For example, the binding affinity is $10^{-6}$ M to $10^{-12}$ M, $10^{-7}$ M to $10^{-12}$ M, $10^{-8}$ M to $10^{-12}$ M, $10^{-9}$ M to $10^{-12}$ M, $10^{-5}$ M to $10^{-11}$ M, $10^{-6}$ M to $10^{-11}$ M, $10^{-7}$ M to $10^{-11}$ M, $10^{-8}$ M to $10^{-11}$ M, $10^{-9}$ M to $10^{-11}$ M, $10^{-10}$ M to $10^{-11}$ M, $10^{-5}$ M to $10^{-10}$ M, $10^{-6}$ M to $10^{-10}$ M, $10^{-7}$ M to $10^{-10}$ M, $10^{-8}$ M to $10^{-10}$ M, $10^{-9}$ M to $10^{-10}$ M, $10^{-5}$ M to $10^{-9}$ M, $10^{-6}$ M to $10^{-9}$ M, $10^{-7}$ M to $10^{-9}$ M, $10^{-8}$ M to $10^{-9}$ M, $10^{-5}$ M to $10^{-8}$ M, $10^{-6}$ M to $10^{-8}$ M, $10^{-7}$ M to $10^{-8}$ M, $10^{-5}$ M to $10^{-7}$ M, $10^{-6}$ M to $10^{-7}$ M or $10^{-5}$ M to $10^{-6}$ M.

In one embodiment of the present invention, a library may be constructed to improve the affinity of the CDR region of an antibody that specifically binds to IL-5Rα and may be realized by a method including (1) selecting an amino acid site having a high possibility of binding to IL-5Rα, among six complementary binding sites (CDRs) involved in antigen-binding of light-chain variable regions (VL) and heavy-chain variable regions (VH) of hu2B7 and 5R65 antibodies as library templates, (2) designing a degenerated codon primer and a spiked oligonucleotide capable of encoding an amino acid to be included in the library at the selected amino acid site, and (3) expressing the designed heavy-chain and light-chain variable region libraries in the form of scFab or Fab using a yeast surface expression system.

In one embodiment of the present invention, screening may be performed using the library to isolate the antibody scFab that specifically binds to sIL-5Rα to improve affinity thereof.

The method for screening the antibody that specifically binds to sIL-5Rα according to the present invention may be carried out by a method including:

(1) expressing an antibody scFab library capable of binding to sIL-5Rα using a yeast surface expression system;
(2) constructing and expressing an sIL-5Rα vector fused with a Hit Tag;
(3) binding sIL-5Rα to the library and selecting the yeast maintaining to bind to sIL-5Rα even after the conditions for dissociating the bound sIL-5Rα are satisfied (kinetic screening); and
(4) measuring the affinity of the binding between sIL-5Rα and the library.

As described above, the anti-IL-5Rα antibody according to the present invention is an antibody binding with high affinity to sIL-5Rα, which is obtained by selecting antibodies to sIL-5Rα from the human antibody scFab library expressed on the yeast cell surface, additionally constructing an antibody Fab library on the yeast surface to improve affinity, and selecting an antibody binding with high affinity to sIL-5Rα through kinetic screening.

Techniques for identifying and separating high-affinity antibodies from libraries are important for the separation of new therapeutic antibodies. The separation of high-affinity antibodies from libraries may depend on the size of the libraries, the production efficiency in bacterial cells, and the variety of libraries. The size of the libraries is reduced by improper folding of the antibody- or antigen-binding protein and inefficient production due to the presence of the stop codon. Expression in bacterial cells can be inhibited when the antibody- or antigen-binding domain is not properly folded. Expression can be improved by alternately mutating residues on the surface of the variable/constant interfaces or the selected CDR residues.

It is important to generate various libraries of antibody- or antigen-binding proteins in the separation of high-affinity antibodies. CDR3 regions have often been found to participate in antigen binding. Since the CDR3 region on the heavy-chain varies considerably in terms of size, sequence and structurally dimensional morphology, various libraries can be prepared using the same.

Also, diversity can be created by randomizing the CDR regions of variable heavy- and light-chains using all 20 amino acids at each position. The use of all 20 amino acids results in antibody sequences having increased diversity and an increased chance of identifying new antibodies.

The non-human (e.g., murine) antibody of the "humanized" form is a chimeric antibody containing a minimal sequence derived from non-human immunoglobulin. In most cases, the humanized antibody is a human immunoglobulin (receptor antibody) in which a residue from the hypervariable region of a receptor is replaced with a residue from the hypervariable region of a non-human species (donor antibody) such as a mouse, rat, rabbit or non-human primate having the desired specificity, affinity and ability.

The term "human antibody" means a molecule derived from human immunoglobulin, wherein the entire amino acid sequence constituting the antibody including a complementarity-determining region and a structural region are composed of human immunoglobulin.

A part of the heavy-chain and/or light-chain is identical to or homologous with the corresponding sequence in an antibody derived from a particular species or belonging to a particular antibody class or subclass, while the other chain(s) include "chimeric" antibodies (immunoglobulins) which are identical to or homologous with corresponding sequences in an antibody derived from another species or belonging to another antibody class or subclass, as well as fragments of such antibody exhibiting the desired biological activity.

As used herein, the term "antibody variable region" refers to the light- and heavy-chain regions of an antibody molecule including the amino acid sequences of a complementarity-determining region (CDR; i.e., CDR1, CDR2, and CDR3) and a framework region (FR). VH refers to a variable domain of the heavy-chain. VL refers to a variable domain of the light-chain.

The term "complementarity-determining region" (CDR; i.e., CDR1, CDR2, and CDR3) refers to an amino acid residue of the antibody variable domain that is necessary for antigen binding. Each variable domain typically has three CDR regions, identified as CDR1, CDR2, and CDR3.

In the present invention, the antibody or antigen-binding fragment thereof binding to IL-5Rα may include a heavy-chain CDR1 of SEQ ID NO: 3, a heavy-chain CDR2 of SEQ ID NO: 4, and a heavy-chain CDR3 of SEQ ID NO: 5, and a light-chain CDR1 of SEQ ID NO: 8, a light-chain CDR2 of SEQ ID NO: 9, and a light-chain CDR3 SEQ ID NO: 10,

> a heavy-chain CDR1 of SEQ ID NO: 3, a heavy-chain CDR2 of SEQ ID NO: 15, a heavy-chain CDR3 of SEQ ID NO: 5 and a light-chain CDR1 of SEQ ID NO: 8, a light-chain CDR2 of SEQ ID NO: 9, and a light-chain CDR3 of SEQ ID NO: 10;
> a heavy-chain CDR1 of SEQ ID NO: 3, a heavy-chain CDR2 of SEQ ID NO: 16, a heavy-chain CDR3 of SEQ ID NO: 5 and a light-chain CDR1 of SEQ ID NO: 8, a light-chain CDR2 of SEQ ID NO: 9, and a light-chain CDR3 of SEQ ID NO: 10;
> a heavy-chain CDR1 of SEQ ID NO: 3, a heavy-chain CDR2 of SEQ ID NO: 17, a heavy-chain CDR3 of SEQ ID NO: 5 and a light-chain CDR1 of SEQ ID NO: 8, a light-chain CDR2 of SEQ ID NO: 9, and a light-chain CDR3 of SEQ ID NO: 10;
> a heavy-chain CDR1 of SEQ ID NO: 3, a heavy-chain CDR2 of SEQ ID NO: 18, a heavy-chain CDR3 of SEQ ID NO: 5 and a light-chain CDR1 of SEQ ID NO: 8, a light-chain CDR2 of SEQ ID NO: 9, and a light-chain CDR3 of SEQ ID NO: 10;
> a heavy-chain CDR1 of SEQ ID NO: 3, a heavy-chain CDR2 of SEQ ID NO: 15, a heavy-chain CDR3 of SEQ ID NO: 27 and a light-chain CDR1 of SEQ ID NO: 8, a light-chain CDR2 of SEQ ID NO: 9, and a light-chain CDR3 of SEQ ID NO: 10;
> a heavy-chain CDR1 of SEQ ID NO: 3, a heavy-chain CDR2 of SEQ ID NO: 15, a heavy-chain CDR3 of SEQ ID NO: 28 and a light-chain CDR1 of SEQ ID NO: 8, a light-chain CDR2 of SEQ ID NO: 9, and a light-chain CDR3 of SEQ ID NO: 10;
> a heavy-chain CDR1 of SEQ ID NO: 3, a heavy-chain CDR2 of SEQ ID NO: 15, a heavy-chain CDR3 of SEQ ID NO: 29 and a light-chain CDR1 of SEQ ID NO: 8, a light-chain CDR2 of SEQ ID NO: 9, and a light-chain CDR3 of SEQ ID NO: 10;
> a heavy-chain CDR1 of SEQ ID NO: 3, a heavy-chain CDR2 of SEQ ID NO: 15, a heavy-chain CDR3 of SEQ ID NO: 30 and a light-chain CDR1 of SEQ ID NO: 8, a light-chain CDR2 of SEQ ID NO: 9, and a light-chain CDR3 of SEQ ID NO: 10;
> a heavy-chain CDR1 of SEQ ID NO: 3, a heavy-chain CDR2 of SEQ ID NO: 15, a heavy-chain CDR3 of SEQ ID NO: 31 and a light-chain CDR1 of SEQ ID NO: 8, a light-chain CDR2 of SEQ ID NO: 9, and a light-chain CDR3 of SEQ ID NO: 10; or
> a heavy-chain CDR1 of SEQ ID NO: 3, a heavy-chain CDR2 of SEQ ID NO: 15, a heavy-chain CDR3 of SEQ ID NO: 32 and a light-chain CDR1 of SEQ ID NO: 8, a light-chain CDR2 of SEQ ID NO: 9, and a light-chain CDR3 of SEQ ID NO: 10.

Specifically, the antibody according to the present invention may include the heavy-chain and light-chain CDR sequences shown in Tables 1, 3, 6 and 11.

The term "framework region" (FR) refers to a variable domain residue other than a CDR residue. Each variable domain typically has four FRs, identified as FR1, FR2, FR3, and FR4.

The antibody or antigen-binding fragment thereof binding to IL-5Rα may include a heavy-chain variable region including a sequence selected from the group consisting of SEQ ID NOS: 1 to 2, 11 to 14 and 21 to 26.

The antibody or antigen-binding fragment thereof binding to the extracellular domain of IL-5Rα may include a light-chain variable region including a sequence selected from the group consisting of SEQ ID NOS: 6 to 7.

In a specific embodiment of the present invention, the antibody or antigen-binding fragment thereof may include the following:

a heavy-chain variable region of SEQ ID NO: 1 and a light-chain variable region of SEQ ID NO: 6;

a heavy-chain variable region of SEQ ID NO: 2 and a light-chain variable region of SEQ ID NO: 7;

a heavy-chain variable region of SEQ ID NO: 11 and a light-chain variable region of SEQ ID NO: 7;

a heavy-chain variable region of SEQ ID NO: 12 and a light-chain variable region of SEQ ID NO: 7;

a heavy-chain variable region of SEQ ID NO: 13 and a light-chain variable region of SEQ ID NO: 7;

a heavy-chain variable region of SEQ ID NO: 14 and a light-chain variable region of SEQ ID NO: 7;

a heavy-chain variable region of SEQ ID NO: 21 and a light-chain variable region of SEQ ID NO: 7;

a heavy-chain variable region of SEQ ID NO: 22 and a light-chain variable region of SEQ ID NO: 7;

a heavy-chain variable region of SEQ ID NO: 23 and a light-chain variable region of SEQ ID NO: 7;

a heavy-chain variable region of SEQ ID NO: 24 and a light-chain variable region of SEQ ID NO: 7;

a heavy-chain variable region of SEQ ID NO: 25 and a light-chain variable region of SEQ ID NO: 7; or

a heavy-chain variable region of SEQ ID NO: 26 and a light-chain variable region of SEQ ID NO: 7.

Specifically, the antibody according to the present invention may include the heavy-chain and light-chain CDR sequences shown in Tables 2, 4, 7 and 12.

In addition, the anti-IL-5Rα antibody or antigen-binding fragment thereof according to the present invention also includes an antibody or antigen-binding fragment thereof in which a part of the amino acid sequence is substituted in the anti-IL-5Rα antibody or antigen-binding fragment thereof according to the present invention through conservative substitution.

As used herein, the term "conservative substitution" refers to modifications of polypeptides that involve the substitution of one or more amino acids with other amino acids having similar biochemical properties that do not result in loss of the biological or biochemical function of the polypeptides. The term "conservative amino acid substitution" refers to substitution of the amino acid residue with an amino acid residue having a similar side chain. Families of amino acid residues having similar side chains have been defined and are well known in the art to which the present invention pertains. These families include amino acids with basic side chains (e.g., lysine, arginine and histidine), amino acids with acidic side chains (e.g., aspartic acid and glutamic acid), amino acids with uncharged polar side chains (e.g., glycine, asparagine, glutamine, serine, threonine, tyrosine, and cysteine), amino acids with nonpolar side chains (e.g., alanine, valine, leucine, isoleucine, proline, phenylalanine, methionine, and tryptophan), amino acids with beta-branched side chains (e.g., threonine, valine, and isoleucine), and amino acids with aromatic side chains (e.g., tyrosine, phenylalanine, tryptophan, and histidine). It is found that the antibody according to the present invention retains the activity thereof despite having conservative amino acid substitutions.

The antibody or antibody-binding fragment thereof according to the present invention may include not only an antibody but also biological equivalents thereto, as long as it can specifically recognize an antigen protein. For example, additional variations can be made to the amino acid sequence of the antibody in order to further improve the binding affinity and/or other biological properties of the antibody. Such variations include, for example, deletion, insertion and/or substitution of the amino acid sequence residues of the antibody. Such amino acid mutations are based on the relative similarity of amino-acid side-chain substituents, such as the hydrophobicity, hydrophilicity, charge and size thereof. It can be seen through analysis of the size, shape and type of amino-acid side-chain substituents that all of arginine, lysine and histidine are positively charged residues; alanine, glycine and serine have similar sizes; and phenylalanine, tryptophan and tyrosine have similar shapes. Thus, based on these considerations, arginine, lysine and histidine; alanine, glycine and serine; and phenylalanine, tryptophan and tyrosine are considered to be biologically functional equivalents.

When taking into consideration mutations having biologically equivalent activity, the antibody or a nucleotide molecule encoding the same according to the present invention is interpreted to include a sequence having substantial identity with the sequence set forth in the sequence number. The term "substantial identity" means that a sequence has a homology of at least 90%, preferably a homology of at least 90%, most preferably at least 95%, at least 96%, at least 97%, at least 98%, and at least 99%, when aligning the sequence of the present invention and any other sequence so as to correspond to each other as much as possible and analyzing the aligned sequence using algorithms commonly used in the art. Alignment methods for sequence comparison are well-known in the art. The NCBI Basic Local Alignment Search Tool (BLAST) is accessible through NCBI or the like, and can be used in conjunction with sequence analysis programs such as BLASTP, BLASTM, BLASTX, TBLASTN and TBLASTX over the Internet. BLAST is available at www.ncbi.nlm.nih.gov/BLAST/. A method of comparing sequence homology using this program can be found at www.ncbi.nlm.nih.gov/BLAST/blast help.html.

Based on this, the antibody or antigen-binding fragment thereof according to the present invention can have a homology of 90%, 91%, 92%, 93%, 94%, 95%, 96%, 97%, 98%, 99%, or more compared to the sequence disclosed herein or the entirety thereof. Homology can be determined through sequence comparison and/or alignment by methods known in the art. For example, the percentage sequence homology of the nucleic acid or protein according to the present invention can be determined using a sequence comparison algorithm (i.e., BLAST or BLAST 2.0), manual alignment, or visual inspection.

In another aspect, the present invention is directed to a nucleic acid encoding the antibody or an antigen-binding fragment thereof. The antibody or antigen-binding fragment thereof can be produced in a recombinant manner by isolating the nucleic acid encoding the antibody or antigen-binding fragment thereof of the present invention.

The term "nucleic acid" is intended to encompass both DNA (gDNA and cDNA) and RNA molecules, and a nucleotide, which is a basic constituent unit of a nucleic acid, includes naturally derived nucleotides as well as analogues, wherein sugar or base moieties are modified. The sequence of the nucleic acid encoding heavy- and light-chain variable regions of the present invention can vary. Such variation includes addition, deletion, or non-conservative or conservative substitution of nucleotides.

The DNA encoding the antibody can be easily separated or synthesized using conventional molecular biological techniques (for example, using an oligonucleotide probe capable of specifically binding to DNA encoding heavy and light-chains of the antibody). Nucleic acids are isolated and inserted into replicable vectors for further cloning (amplification of DNA) or further expression. Based on this, in another aspect, the present invention is directed to a recombinant expression vector including the nucleic acid.

As used herein, the term "vector" refers to a means for expressing target genes in host cells, and includes plasmid vectors, cosmid vectors, and viral vectors such as bacteriophage vectors, adenovirus vectors, retroviral vectors and adeno-associated viral vectors. Vector components generally include, but are not limited to, one or more of the following components: signal sequences, replication origins, one or more antibiotic resistance marker genes, enhancer elements, promoters, and transcription termination sequences. The nucleic acid encoding the antibody is operably linked to promoters, transcription termination sequences or the like.

The term "operably linked" means a functional linkage between a nucleic acid expression regulation sequence (e.g., an array of promoter, signal sequence or transcription regulator binding sites) and another nucleic acid sequence, and enables the regulation sequence to regulate the transcription and/or translation of the other nucleic acid sequence.

When a prokaryotic cell is used as a host, it generally includes a potent promoter capable of conducting transcription (such as a tac promoter, a lac promoter, a lacUV5 promoter, a lpp promoter, a pLλ promoter, a pRλ promoter, a rac5 promoter, an amp promoter, a recA promoter, SP6 promoter, a trp promoter, or a T7 promoter), a ribosome-binding site for initiation of translation, and a transcription/translation termination sequence. In addition, for example, when a eukaryotic cell is used as a host, it includes a promoter (*e.g.*, a metallothionein promoter, a β-actin promoter, a human hemoglobin promoter and a human muscle creatine promoter) derived from the genome of mammalian cells, or a promoter derived from a mammalian virus such as an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter, and generally has a polyadenylation sequence as a transcription termination sequence.

Optionally, the vector may be fused with another sequence in order to facilitate purification of the antibody expressed therefrom. The sequence to be fused therewith includes, for example, glutathione S-transferase (Pharmacia, USA), maltose-binding protein (NEB, USA), FLAG (IBI, USA), 6x His (hexahistidine; Qiagen, USA) and the like.

The vector includes antibiotic-resistance genes commonly used in the art as selectable markers, and examples thereof include genes conferring resistance to ampicillin, gentamycin, carbenicillin, chloramphenicol, streptomycin, kanamycin, geneticin, neomycin and tetracycline.

In another aspect, the present invention is directed to a cell transfected with the recombinant expression vector. The host cell used to produce the antibody of the present invention may be a prokaryote, yeast or higher eukaryotic cell, but is not limited thereto.

Prokaryotic host cells such as *Escherichia coli,* the genus *Bacillus,* such as *Bacillus subtilis* and *Bacillus thuringiensis, Streptomyces* spp., *Pseudomonas* spp. (for example, *Pseudomonas putida*)*, Proteus mirabilis* and *Staphylococcus* spp. (for example, *Staphylococcus carnosus*) can be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing an antibody or antigen-binding fragment thereof including culturing the host cells to produce an antibody, and isolating the produced antibody from the cultured cells, followed by purification.

Specifically, the method of producing an antibody or antigen-binding fragment thereof binding to human IL-5Rα includes, but is not limited to:

(a) culturing host cells expressing the antibody or antigen-binding fragment thereof according to the present invention to produce the antibody or antigen-binding fragment thereof binding to human IL-5Rα according to the present invention; and
(b) recovering the produced antibody or antigen-binding fragment thereof.

The host cells can be cultured in various media. Any commercially available medium can be used as a culture medium without limitation. All other essential supplements well-known to those skilled in the art may be included in appropriate concentrations. Culture conditions such as temperature and pH are those that are conventionally used with the host cells selected for expression, which will be apparent to those skilled in the art.

The recovery of the antibody or antigen-binding fragment thereof can be carried out, for example, by centrifugation or ultrafiltration to remove impurities and further purification of the resulting product using, for example, affinity chromatography. Other additional purification techniques such as anion or cation exchange chromatography, hydrophobic interaction chromatography and hydroxyapatite (HA) chromatography may be used.

In another aspect, the present invention is directed to a conjugate in which the antibody or antigen-binding fragment thereof is fused with a bioactive molecule selected from the group consisting of peptides, proteins, small-molecule drugs, nucleic acids, nanoparticles and liposomes.

The proteins include antibodies, fragments of antibodies, immunoglobulins, peptides, enzymes, growth factors, cytokines, transcription factors, toxins, antigenic peptides, hormones, transport proteins, motor function proteins, receptors, signaling proteins, storage proteins, membrane proteins, transmembrane proteins, internal proteins, external proteins, secreted proteins, viral proteins, sugar proteins, truncated proteins, protein complexes, chemically modified proteins and the like.

The term "small-molecule drugs" refers to an organic compound, an inorganic compound or an organometallic compound that has a molecular weight of less than about 1,000 Da and has activity as a therapeutic agent for diseases, which is widely used herein. The small-molecule drug used herein includes oligopeptides and other biomolecules having a molecular weight of less than about 1,000 Da.

As used herein, the term "nanoparticle" refers to a particle including a material having a diameter of 1 to 1,000 nm, and the nanoparticle may be a metal/metal core-shell complex including a metal nanoparticle, a metal nanoparticle core and a metal shell including the core, a metal/non-metal core-shell complex including a metal nanoparticle core and a non-metal shell surrounding the core, or a nonmetal/metal core-shell complex including a nonmetal nanoparticle core and a metal shell surrounding the core. According to one embodiment, the metal may be selected from gold, silver, copper, aluminum, nickel, palladium, platinum, magnetic iron, and oxides thereof, but is not limited thereto, and the nonmetal may be selected from silica, polystyrene, latex and acrylic substances, but is not limited thereto.

The liposome consists of one or more lipid bilayer membranes surrounding an aqueous internal compartment that can self-associate. Liposomes can be specified based on the type and size of the membrane thereof. Small unilamellar vesicles (SUVs) have a single membrane, and may have a diameter of 20 nm to 50 nm. Large unilamellar vesicles (LUV) may have a diameter of 50 nm or more. Oligolamellar large vesicles and multilamellar large vesicles have multiple, generally concentric, membrane layers, and may be 100 nm or more in diameter. Liposomes having a plurality of non-concentric membranes, that is, several small vesicles contained within larger vesicles, are called "multivesicular vesicles".

As used herein, the term "fusion" refers to the integration of two molecules having different or identical functions or structures, and includes fusion through any physical, chemical or biological method capable of binding the antibody or antigen-binding fragment thereof to the protein, small-molecule drug, nanoparticle, or liposome. The fusion may preferably be carried out using a linker peptide, and the linker peptide may mediate the fusion with the bioactive molecule at various positions of the antibody light-chain variable region, antibody, or fragment thereof according to the present invention.

As used herein, the term "effector function" refers to the type of biological activity associated with the Fc region of an antibody (wild-type sequence of the Fc region or a variant of the amino acid sequence of the Fc region) and depends on the isotype of the antibody. Examples of antibody effector functions include C1q binding, complement dependent cytotoxicity (CDC); Fc receptor binding, antibody dependent cell-mediated cytotoxicity (ADCC), phagocytosis, downregulation of cell surface receptors (e.g., B-cell receptor, BCR) and B-cell activation.

The term "antibody-dependent cellular cytotoxicity" or "ADCC" refers to a reaction in which effector cells (e.g., T-cells and NK-cells) lyse target cells labeled by a specific antibody. ADCC is also independent of the immune complement system, which lyses the target, but does not require other cells. ADCC typically requires effector cells known to be natural killer (NK) cells that interact with immunoglobulin G (IgG) antibodies. However, macrophages, neutrophils and eosinophils can also mediate ADCC, for example, eosinophils that kill certain parasitic worms known as parasites via IgE antibodies.

The bioactive molecule that can be conjugated to the antibody or antigen-binding fragment thereof according to the present invention is a small molecule drug, particularly preferably a drug that is effective in the treatment of allergic diseases, inflammatory diseases, and/or diseases caused by an increase in eosinophils, for example, hypereosinophilic syndrome (HES), hypereosinophilia, asthma including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, rheumatoid arthritis, and the like.

More preferably, examples of the bioactive molecule include, but are not limited to, beta agonists such as indacaterol, formoterol, vilanterol, albuterol, levalbuterol, and theophylline, anticholinergic agents such as ipratropium, tiotropium, and glycopyrrolate, and leukotriene modifiers such as montelukast, zafirlukast, and zileuton.

In another aspect, the present invention is directed to a bispecific antibody or multispecific antibody including the antibody or antigen-binding fragment thereof.

As used herein, the term "bispecific antibody" refers to a protein capable of binding to two different types of antigens (target proteins). Specifically, the bispecific antibody does not exist naturally and is preferably prepared by genetic engineering or any method. The term "bispecific antibody" of the present invention may be used interchangeably with "bitarget antibody", "biantibody" or "biantibody protein".

The bispecific antibody refers to a molecule that has an antigen-binding site linking directly or via a linker or forms a heterodimer through electrostatic interaction.

The term "valent" means the presence of a specified number of binding sites specific to an antigen in the molecule. As such, the terms "monovalent", "bivalent", "tetravalent", and "hexavalent" refer to the presence of each of 1, 2, 4, and 6 binding sites specific for an antigen in a molecule.

The term "multispecific antibody" refers to an antibody that has binding specificity for at least three different antigens. The multispecific antibody includes a tri- or higher antibody, for example, a trispecific antibody, a tetraspecific antibody, or an antibody that targets more targets.

Any antibody may be used without limitation as the antibody capable of forming a bispecific antibody or a multispecific antibody together with the antibody according to the present invention as long as it is used in combination with the anti-IL-5Rα antibody according to the present invention to provide synergistically therapeutic or prophylactic effects for diseases caused by an increase in eosinophils, for example, hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, and rheumatoid arthritis.

For example, suitable antibodies include, but are not limited to, anti-IgE antibodies such as omalizumab and ligelizumab, anti-interleukin or anti-interleukin receptor antibodies, such as anti-IL4R antibodies.

In another aspect, the present invention is directed to a pharmaceutical composition for preventing or treating allergic diseases, inflammatory diseases, and/or diseases caused by an increase in eosinophils, especially, diseases caused by an increase in eosinophils, containing the antibody or antigen-binding fragment thereof as an active ingredient.

Examples of diseases that can be treated using the antibody according to the present invention include, but are not limited to, hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, and rheumatoid arthritis.

In another aspect, the present invention is directed to a pharmaceutical composition for preventing or treating allergic diseases, inflammatory diseases, and/or diseases caused by an increase in eosinophils, including (a) a pharmaceutically effective amount of the antibody or antigen-binding fragment thereof according to the present invention, and (b) a pharmaceutically acceptable carrier.

The present invention is also directed to a method for preventing or treating allergic diseases, inflammatory diseases and/or diseases caused by an increase in eosinophils, including administering the antibody or antigen-binding fragment thereof according to the present invention in an effective amount required for a patient.

The antibody according to the present invention is useful for the prevention or treatment of IL-5-mediated diseases by removing, inhibiting, or reducing IL-5 activity. The antibody according to the present invention binds to IL-5R$\alpha$ and is used for the treatment of diseases associated with IL-5 activity. Examples of the diseases include eosinophilic asthma, chronic obstructive pulmonary disease (COPD), Churg-Strauss syndrome, eosinophilic esophagitis, eosinophilic gastroenteritis or heavy-chain diseases.

In order to treat the autoimmune disease or related autoimmune condition, the anti-IL-5R$\alpha$ antibody of the present invention can be administered to a patient in combination with other therapeutic agents using multi-drug therapy. The anti-IL-5R$\alpha$ antibody or antigen-binding fragment thereof according to the present invention may be administered simultaneously with, sequentially or alternately with immunosuppressive agents, or after resistance to other therapies appears. Immunosuppressive agents may be administered in an amount identical to or lower than that used in the art. The selection of preferred immunosuppressive agent may depend on many factors, including the type of disease to be treated and the patient's medical history.

As used herein, the term "prevention" refers to any action causing the suppression of growth of allergic diseases, inflammatory diseases, and/or diseases caused by an increase in eosinophils or the delay of progression of such diseases by administration of the composition according to the present invention. The term "treatment" means suppression of the progression of allergic diseases, inflammatory diseases, and/or diseases caused by an increase in eosinophils or alleviation or elimination of such diseases. The antibodies of the present invention can be useful both *in vitro* and *in vivo* for applications involving cells expressing IL-5R$\alpha$.

The pharmaceutical composition of the present invention contains the antibody or antigen-binding fragment thereof or the conjugate according to the present invention, and the pharmaceutical composition may further contain a pharmaceutically acceptable carrier, in addition to the component for administration of the pharmaceutical composition of the present invention. The term "pharmaceutically acceptable carrier" as used herein refers to a carrier or diluent that does not impair the biological activities or properties of the administered compound and does not stimulate an organism. Pharmaceutically acceptable carriers for compositions that are formulated into liquid solutions are sterilized and biocompatible, and examples thereof include saline, sterile water, buffered saline, albumin injection solutions, dextrose solutions, maltodextrin solutions, glycerol, and mixtures of one or more thereof. If necessary, other conventional additives such as antioxidants, buffers and bacteriostatic agents may be added. In addition, diluents, dispersants, surfactants, binders and lubricants can be additionally added to formulate injectable solutions such as aqueous solutions, suspensions and emulsions, pills, capsules, granules, or tablets.

The pharmaceutical composition according to the present invention may be any one of various oral or parenteral formulations. In this regard, the pharmaceutical composition may be formulated using an ordinary diluent or excipient such as a filler, a thickener, a binder, a wetting agent, a disintegrant, a surfactant, or the like. Solid formulations for oral administration may include tablets, pills, powders, granules, capsules and the like. Such a solid formulation is prepared by mixing at least one compound with at least one excipient such as starch, calcium carbonate, sucrose, lactose or gelatin. In addition to a simple excipient, a lubricant such as magnesium stearate or talc may be further used. Liquid formulations for oral administration may include suspensions, solutions for internal use, emulsions, syrups, and the like. In addition to a simple diluent such as water or liquid paraffin, various excipients such as wetting agents, sweeteners, aromatics and preservatives may be incorporated in the liquid formulations. In addition, formulations for parenteral administration include sterile aqueous solutions, non-aqueous solvents, suspensions, emulsions, lyophilizates, suppositories and the like. Useful non-aqueous solvents and suspensions include propylene glycol, polyethylene glycol, vegetable oils such as olive oil and injectable esters such as ethyl oleate. The base ingredients of suppositories include Witepsol, macrogol, Tween 61, cacao butter, laurin butter and glycerogelatin.

The method for treating inflammatory diseases using the antibody or antigen-binding fragment thereof or the conjugate according to the present invention includes administering, to a subject, a pharmaceutically effective amount of the antibody or antigen-binding fragment thereof, or the conjugate. It will be apparent to those skilled in the art that an appropriate total daily dose can be determined based on the judgment of a medical specialist. In addition, the antibody, antigen-binding fragment thereof, or the conjugate may be administered in a single dose, or may be divided into multiple doses. However, in consideration of the objects of the present invention, the specific therapeutically effective amount for a certain patient is preferably determined depending upon a variety of factors, including the type and extent of the response to be achieved, as well as the presence of other agents used, the specific composition, the age, body weight, general state of health, gender, and diet of the patient, the administration time, the administration route, the treatment period, and drugs used in conjunction with or concurrently with the specific composition, and other similar factors well-known in the field of pharmaceuticals.

The subject to which the composition of the present invention is administered includes mammals including humans, without limitation thereto.

As used herein, the term "administration" refers to an action of supplying the pharmaceutical composition according to the present invention to a patient by any appropriate method, and the composition according to the present invention may be orally or parenterally administered through any one of various routes enabling the composition to be delivered to a target tissue.

The antibody or antigen-binding fragment thereof according to the present invention may be used as a single drug or in combination with a conventional therapeutic agent.

Any drug may be used without limitation as the drug that can be used in combination therapy with the antibody according to the present invention so long as it can be used to treat diseases caused by an increase in eosinophils, for example, hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophiliamyalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, and rheumatoid arthritis.

Preferably, examples of the drug include, but are not limited to, beta agonists such as indacaterol, formoterol, vilanterol, albuterol, levalbuterol, and theophylline, anticholinergic agents such as ipratropium, tiotropium, and glycopyrrolate, leukotriene modifiers such as montelukast, zafirlukast, and zileuton, inhaled corticosteroids such as fluticasone propionate, budesonide, ciclesonide, beclomethasone, and mometasone, and anti-IgE antibodies such as omalizumab and ligelizumab.

In addition, the present invention is directed to a method for treating a disease including administering the anti-IL-5R$\alpha$ antibody or antigen-binding fragment thereof according to the present invention to a patient in need of treatment.

In another aspect, the present invention is directed to a composition for diagnosing allergic diseases, inflammatory diseases, and/or diseases caused by an increase in eosinophils including the anti-IL-5R$\alpha$ antibody or antigen-binding fragment thereof. In another aspect, the present invention is directed to a kit for diagnosing allergic diseases, inflammatory diseases, and/or diseases caused by an increase in eosinophils containing the composition for diagnosing the diseases.

As used herein, the term "diagnosis" means determining the presence or features of pathophysiology. In the present invention, diagnosis serves to determine the onset or progress of diagnosing allergic diseases, inflammatory diseases, and/or diseases caused by an increase in eosinophils.

For diagnostic methods using the antibody or antigen-binding fragment thereof according to the present invention, the drug may include a detectable label used to detect the presence of IL-4R$\alpha$ antigen-expressing cells *in vitro* or *in vivo.* Radioisotopes that are detectable *in vivo* such as labels that can be detected using scintillation, magnetic resonance imaging or ultrasound can be used for clinical diagnostic applications. Useful scintillation labels include positron emitters and $\gamma$-emitters. Representative contrast agents as magnetic sources for imaging include paramagnetic or superparamagnetic ions (e.g., iron, copper, manganese, chromium, erbium, europium, dysprosium, holmium and gadolinium), iron oxide particles, and water-soluble contrast agents. For ultrasonic detection, a gas or liquid can be trapped in the porous inorganic particles released as a microbubble contrast agent. Detectable labels useful for *in-vitro* detection include fluorophores, detectable epitopes or binders and radiolabels.

The kit for diagnosing allergic diseases, inflammatory diseases, and/or diseases caused by an increase in eosinophils may further include a composition, solution or device having one or more other components suitable for the analysis method.

In one embodiment, the kit may include a bottle, vial, bag, needle, or syringe. The container may be made from various materials, such as glass, plastic, or metal. The label on the container may provide instructions for use. The kit may further include other materials desirable from commercial and usage perspectives, such as other buffers, diluents, filters, needles and syringes.

Hereinafter, the present invention will be described in more detail with reference to examples. However, it will be obvious to those skilled in the art that these examples are provided only for illustration of the present invention and should not be construed as limiting the scope of the present invention.

Example 1: Expression of recombinant human interleukin-5 receptor α (IL-5Rα)

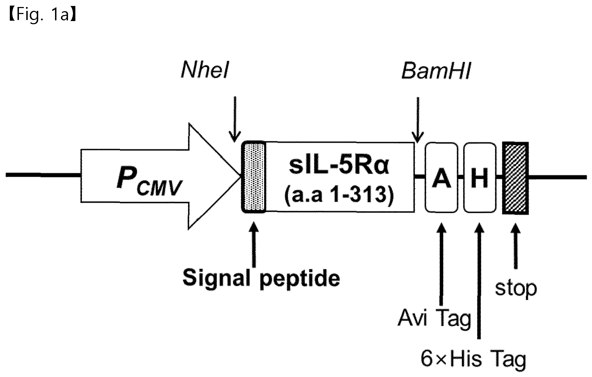

An antigenic protein to obtain an antibody specific to human interleukin-5 receptor α (IL-5Rα) was prepared. Since IL-5Rα is a cell membrane glycoprotein, the amino acid residue Asp1-Asn313 as the extracellular domain of the IL-5Rα sequence represented by SEQ ID NO: 19 was introduced into an animal cell expression vector. The amino acid residue Asp1-Asn313 of the extracellular domain is called "sIL-5Rα". sIL-5Rα (amino acid residues Asp1-Asn313) was cloned into an animal expression vector (pSecTag2A) using restriction enzymes NheI/BamHI, and an Avi tag (amino acid sequence; GLNDIFEAQKIEWHE) peptide and 6× histidine (6 ×His) labeled peptide were fused to the C-terminus to construct pSecTag2A-sIL-5Rα (FIG. 1A). To express and purify the antigenic protein, HEK293F (Invitrogen) cells were transiently transfected. For transfection of 100 mL of the cells in a shake flask (Corning), HEK293F cells were seeded at a density of $1.0 \times 10^6$ cells/ml in a medium and incubated at 120 rpm, 8% $CO_2$ and 37°C. A total of 125 μg of the constructed plasmid was diluted in 5 ml serum-free Freestyle 293 expression medium (Invitrogen), filtered, mixed with 5 ml of a medium diluted with 375 μg of polyethylenimine (PEI) and then was reacted for 10 minutes at room temperature. Then, the reacted mixed medium was injected into the 90 ml previously seeded cells, followed by incubation at 120 rpm and 8% $CO_2$ and further incubation for 6 days. Proteins were purified from the cell incubation supernatant collected with reference to standard protocols. The supernatant was applied to a nickel Sepharose column (Ni Sepharose™ 6 Fast Flow, GE Healthcare) and washed with a washing buffer (50 mM phosphate, 300 mM NaCl, 30 mM imidazole pH 8.0), and the protein was eluted with an elution buffer (50 mM phosphate, 300 mM NaCl, 250 mM imidazole, pH 8.0).

The eluted protein was concentrated using a Vivaspin 10,000 MWCO (Sartorius) centrifugal concentrator after exchanging the buffer with a storage buffer (PBS, pH 6.5) . The absorbance of the purified protein at a wavelength of 562 nm was measured and the amount thereof was quantified using a solution in the BCA protein assay kit (Thermo) according to the drawn standard curve.

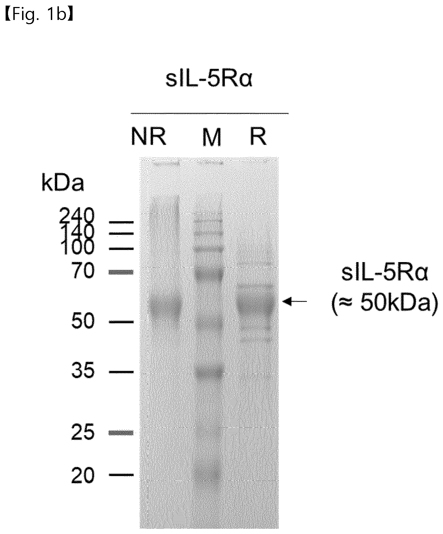

The purity of 3 μg of the purified sIL-5Rα protein was confirmed through SDS-PAGE analysis (FIG. 1B). The purified proteins showed slightly extended bands at a size larger than the predicted molecular weight (37 kDa). The extended bands are due to different glycosylation patterns. There is cysteine (residue 66 in the sequence of IL-5Ra represented by SEQ ID NO: 19) that does not form a disulfide bond in the extracellular domain of IL-5Ra and proteins forming aggregates were observed under non-reducing (NR) conditions.

Example 2: Production of anti-IL-5Rα mouse antibodies through mouse immunization

Mouse immunization was performed by A-Frontier (Seoul, Korea). Briefly, 100 μg of the protein (antigen) obtained above was mixed with complete Freund's adjuvant and the resulting mixture was injected intraperitoneally into female Balb/c mice. After 2 weeks, serum was collected from mouse tails and antibody concentration was measured using ELISA. For secondary boosting, 100 ug of an antigen was again mixed and the mixture was injected into the mice. 1 week later, 100 μg of the antigen was injected to perform final boosting. Then, the cells were extracted from the mice, the tissue was washed with the culture medium, and the cells were separated. Myeloma cells (Sp2/0Ag14) were fused with cells isolated from mice using PEG 1500 (Roche, 10 783 641 001). The fusion cells were cultured using 1×HAT (sigma, H0262) culture medium. After ELISA, cells in positive wells are transferred to 24 wells and cultured. The cells are transferred to a 96-well plate using HT culture medium (Gibco, 11067030) and incubated in a 37°C $CO_2$ incubator. Fusion cells secrete anti-IL-5Rα mouse antibody onto the medium. The binding ability to IL-5Ra was first screened by ELISA using the culture medium and IL-5Ra was transiently expressed in HEK293T, which was double confirmed using flow cytometry. Four monoclonal antibodies were derived by repeating the cloning process until the final clone was identified. The isotype of the antibody was confirmed using a Rapid ELISA mouse mAb isotyping kit (Pierce, 37503). m2B7 and m2H12 have an IgG1 heavy-chain and a kappa light-chain, m9B8 has an IgG2a heavy-chain and a kappa light-chain, and m12F9 has an IgG2b heavy-chain and a kappa light-chain.

Then, the provided hybridoma cells were cultured in RPMI medium and the supernatant was applied to a Protein A Sepharose column and washed with PBS (12 mM phosphate, 137 mM NaCl, 2.7 mM KCl, pH 7.4). The antibody was eluted at pH 3.0 using 0.1 M glycine, 0.5 M NaCl buffer, and then the sample was immediately neutralized using 1 M Tris buffer.

The eluted protein was concentrated using a Vivaspin 30,000 MWCO (Sartorius) centrifugal concentrator after buffer exchange with storage buffer (PBS pH7.4) using a PD-10 desalting column (GE Healthcare). The absorbance of the purified protein was measured at a wavelength of 562 nm using a solution in the BCA protein assay kit (Thermo), and the amount was quantified according to a drawn standard curve.

Example 3: Confirmation of binding ability of anti-IL-5Rα mouse antibody

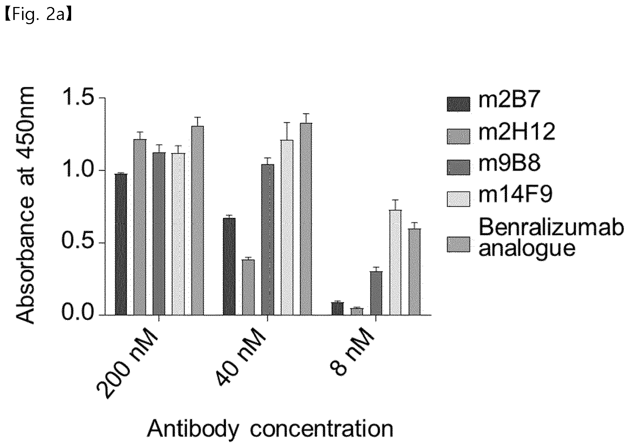

The antigen-binding ability of the antibody was examined using indirect ELISA. The heavy-chain variable region and light-chain variable region (DrugBank Accession No. DB12023) of benralizumab, an anti-IL-5Rα antibody approved by the FDA in 2017, were introduced into the pcDNA3.4 vector encoding the light-chain (CL) and the heavy-chains (CH1, CH2, CH3) of IgG1, which is a commonly used antibody. Benralizumab having an IgG1 constant region was named "benralizumab analogue". The binding ability of the expressed benralizumab analogue was also compared. The sIL-5Rα antigen (Sinobio, 10392-H08H) protein purchased from Synobio was immobilized in a 96-well plate at 50 ng/well at room temperature for 1 hour and blocked with 0.1% PBST (0.1% Tween20, pH 7.4, 137 mM NaCl, 10 mM phosphate, 2.7 mM KCl) for 1 hour at room temperature. After discarding the solution and washing three times with 0.1% PBST, each antibody was sequentially diluted in the range of 8, 40, and 200 nM, and each antibody was added to the blocked plate in an amount of 25 μl/well and reacted at room temperature for 1 hour. After discarding the solution and washing three times with 0.1% PBST, 25 μl of anti-human IgG-HRP antibody (1:8000) or anti-mouse IgG-HRP antibody (1:4000) was added per well as a secondary antibody and reacted at room temperature for 1 hour. After discarding the solution and washing three times with PBST, TMB solution was added in an amount of 25 μl/well, followed by color reaction at room temperature for 1 minute. Then, the reaction was stopped with 2N $H_2SO_4$ and absorbance was measured at 450 nm using an ELISA reader. As can be seen from the results of FIG. 2A, four types of mouse antibodies and benralizumab analogues had a wide range of affinities for sIL-5Rα.

Example 4: Construction of TF-1 cell line expressing IL-5Ra (TF-1/IL-5Rα cells)

To compare the ability of four anti-IL-5Rα mouse antibodies and benralizumab analogues to inhibit IL-5 signal transduction, cell lines were first constructed. After IL-5 binds to IL-5Rα, it mediates signaling by the consensus β receptor (βc receptor). Then, a cell line stably overexpressing IL-5Rα was constructed in the TF-1 cell line expressing the βc receptor. Specifically, the DNA encoding IL-5Ra was cloned into a Lentivirus vector, pLJM1 (Addgene) vector with SalI/EcoRI. $3 \times 10^6$ HEK293T cells were cultured in 10 ml of a medium containing 10% FBS in a cell culture plate and cultured at 5% $CO_2$ and 37°C for 12 hours. When the cells are stabilized, the medium was removed and the plate was washed using DPBS (Wellgene, LB001-04). 40 μl of Lipofectamine 3000 (Invitrogen, USA) was added to 600 μl of Opti-MEM media (Gibco), the constructed lentiviral vectors and viral packaging vectors pMDL, pRSV, and pVSV-G (Addgene) were carefully added thereto for 20 minutes, reaction was performed at room temperature and the result was added to the dish. In addition, 9 ml of DMEM media containing no antibiotic was added thereto, incubated at 37°C, 5% $CO_2$ for 6 hours, and then exchanged with 10 ml of DMEM medium containing 10% FBS, followed by culture for 48 hours. All of the medium in which the lentiviral vector was transiently transfected was filtered and the virus particles in the medium were added to a previously prepared cell culture dish containing TF-1 cells. Antibiotic resistance was measured using a puromycin resistance gene as a selection marker.

Example 5: Comparison of IL-5-dependent proliferation inhibition in cell lines between anti-IL-5Rα mouse antibodies

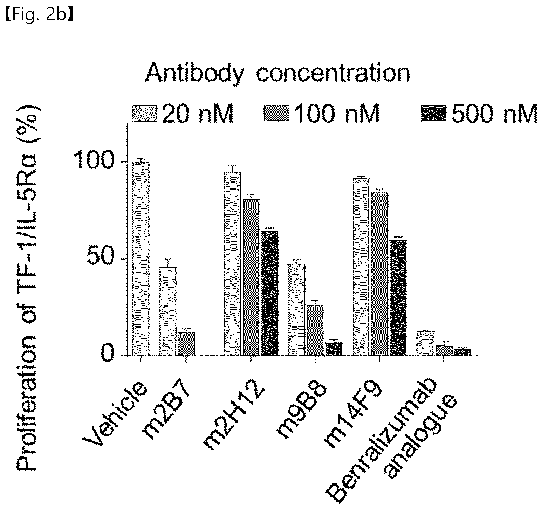

Transformed TF-1 cells stably expressing IL-5Rα (TF-1/IL-5Rα cells) were seeded at 100 μl of $2 \times 10^4$ cells in a 96-well plate. On the same day, 50 μl of 320 pM IL-5 and 50 μl of anti-IL-5Rα mouse antibody solution previously diluted to 80 nM, 400 nM, or 2,000 nM were added and incubated in a 96-well plate at 37°C and 5% $CO_2$ for 40 hours. In order to measure the proliferation capacity of cells, 100 μl of cell suspension was collected from each well, 100 μl of CellTiter-Glo® (manufactured by Promega) was added thereto, reacted at room temperature for 20 minutes and analyzed with a Cytation™ 3 cell imaging multimode reader (FIG. 2B).
The result of the analysis showed that the anti-IL-5Rα mouse antibodies exhibited a lower IL-5 signal blocking effect than the benralizumab analogue, and thereamong, m2B7 exhibited a high IL-5 signal blocking effect.

Example 6: Humanization of anti-IL-5Rα mouse antibody, m2B7

The method commonly used to humanize m2B7 includes comparing the m2B7 antibody framework (FR) with a human antibody framework to select the human antibody framework having the maximum homology and transplanting the heavy- and light-chain CDR (complementary determining regions) sequences of m2B7. Therefore, human germline genes having the highest homology with the heavy-chain variable region gene of m2B7 were analyzed using Ig Blast (https://www.ncbi.nlm.nih.gov/igblast/). The result showed that the heavy-chain variable region had 66.3% homology with the human IGHV1-46*01 gene at the amino acid level and the light-chain variable region had 65.3% homology with human IGKV1-9*01. Since m2B7 had relatively low homology, it had 75.9% and 71.3% homology in the heavy- and light-chain variable regions with daclizumab (anti-IL-2Rα antibody) when compared with the frameworks excluding the CDRs of 37 clinically studied antibodies. Therefore, Kabat Nos. 31-35 (VH-CDR1), 50-65 (VH-CDR2), and 95-102 (VH-CDR3) of mouse antibody m2B7 were defined as heavy-chain variable region CDRs, and Kabat Nos. 24-34 (VL-CDR1), 50-56 (VL-CDR2), and 89-97 (VL-CDR3) were defined as light-chain variable region CDRs and heavy- and light-chain variable region CDRs were introduced into the framework of daclizumab. It is known that direct CDR grafting into human antibody backbone acceptor sequences often results in loss of affinity and specificity for the target antigen. To minimize the possibility of this loss, a residue that plays an important role in forming the loop structure of the CDR must be preserved and this position thereof is referred to as "Vernier zone". Therefore, the 93rd (A→T) amino acid of the heavy-chain variable region in the Vernier zone was back-mutated to the amino acid sequence of the original mouse antibody m2B7. Residues 60 and 70 of the light-chain variable region were also maintained as m2B7 residues, Asp, because Asp occurs preferentially in the human germline sequence. The humanized antibody was named "hu2B7".

Tables 1 and 2 respectively show the heavy-chain CDR sequence and heavy-chain variable region sequences of the anti-IL-5Rα mouse antibody m2B7, and Tables 3 and 4 respectively show the light-chain CDR sequence and light-chain variable region sequence.

[Table 1]

| Heavy-chain variable region name | CDR1 sequence | CDR2 sequence | CDR3 sequence |
|---|---|---|---|
| Kabat No. | 31 32 33 34 35 | 50 51 52 52a 53 54 55 56 57 58 59 60 61 62 63 64 65 | 95 96 97 98 99 100 100a 100b 100c 100d 100e 101 102 |
| m2B7/ hu2B7 | S Y W I N | H I Y P S E S Y T N Y N Q K F K D | D Y Y G R S Y Y Y A M D Y |
|  | SEQ ID No: 3 | SEQ ID No: 4 | SEQ ID No: 5 |

[Table 2]

| Heavy-chain variable region name | sequence | SEQ ID NO: |
|---|---|---|
| m2B7 | QVQLQQPGAELVRPGASVKLSCKASGYTFTSYWINWVKQRPGQGLEWIGHIYPSESYTNYNQKFKDKATLTVDK SSSTAYMQLSSPTSEDSAVYYCTRDYYGRSYYYAMDYWGQGTSVTVSS | SEQ ID No: 1 |
| hu2B7 | QVQLVQSGAEVKKPGSSVKVSCKASGYTFTSYWINWVRQAPGQGLEWIGHIYPSESYTNYNQKFKDKATLTVDK STNTAYMELSSLRSEDTAVYYCTRDYYGRSYYYAMDYWGQGTTLTVSS | SEQ ID No: 2 |

[Table 3]

| Light-chain variable region name | CDR1 sequence | CDR2 sequence | CDR3 sequence |
|---|---|---|---|
| Kabat No. | 24 25 26 27 28 29 30 31 32 33 34 | 50 51 52 53 54 55 56 | 89 90 91 92 93 94 95 96 97 |
| m2B7/ hu2B7 | K A S Q N V G T A V A | W A S T R H T | Q Q F G R Y P Y T |
|  | SEQ ID No: 8 | SEQ ID No: 9 | SEQ ID No: 10 |

[Table 4]

| Light-chain variable region name | sequence | SEQ ID NO: |
|---|---|---|
| m2B7 | DIVMTQSHKFMSTSVGDRVSITCKASQNVGTAVAWYQQKPGQSPKLLIYWASTRHTGVPDRFTGSGSGTDFTFTIS NVQSEDLADYFCQQFGRYPYTFGGGTKLEIK | SEQ ID No: 6 |
| hu2B7 | DIQMTQSPSTLSASVGDRVTITCKASQNVGTAVAWYQQKPGKAPKLLIYWASTRHTGVPDRFSGSGSGTDFTLTIS SLQPDDFATYYCQQFGRYPYTFGSGTKVEVK | SEQ ID No: 7 |

The cell-incubated supernatant obtained by transiently transfecting plasmids encoding the HC and LC of hu2B7 was applied to a protein A Sepharose column, and then washed with PBS (12 mM phosphate, 137 mM NaCl, 2.7 mM KCl, pH 7.4). The antibody was eluted at pH 3.0 using 0.1 M glycine and 0.5 M NaCl buffer, and then the sample was immediately neutralized using 1 M Tris buffer.

The eluted protein was concentrated using a Vivaspin 30,000 MWCO (Sartorius) centrifugal concentrator after buffer exchange with storage buffer (PBS pH 7.4) using a PD-10 desalting column (GE Healthcare). The absorbance of the purified protein was measured at a wavelength of 562 nm using a solution in the BCA protein assay kit (Thermo), and the amount was quantified according to a drawn standard curve.

Example 7: Confirmation of binding ability of anti-IL-5Ra antibodies (m2B7, hu2B7, benralizumab analogues)

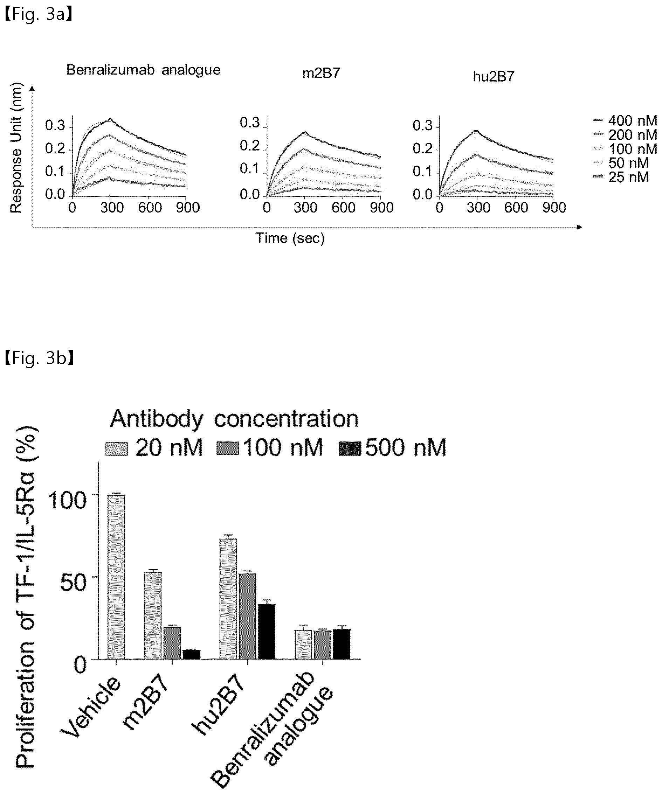

In order to more quantitatively analyze binding ability to sIL-5Rα of m2B7, hu2B7, benralizumab analogues, the binding ability was measured according to the protocol suggested by the manufacturer using an Octet QK$^e$ (ForteBio, USA) system. Specifically, 1x kinetic buffer was prepared by diluting 10x kinetic buffer (ForteBio, 18-1105) in PBS. The antibody was diluted to a concentration of 1 ug/ml in 1x kinetic buffer, the IL-5Ra purified in Example 1 was sequentially diluted at concentrations of 400, 200, 100, 12.5, 6.25 and 0 nM in 1x kinetic buffer, and 200 μl of each dilution was injected into an opaque 96-well plate through which light does not pass. Antigen affinity of antibodies was analyzed through the variation in refractive index occurring when the antibody bound to the antigen was detached therefrom while the AHC (anti-human IgG Fc capture) sensor chip moved in the order of 1x kinetic buffer, antibody dilution solution, 1x kinetic buffer, antigen dilution solution, and 1x kinetic buffer. Affinity was calculated with Octet Data Analysis software 11.0 in a 1:1 binding model. The results are shown in FIG. 3A.

Table 5 shows the results of analysis of affinity of selected anti-IL-5Rα antibodies to IL-5Ra using the Octet QK$^e$ system. The affinity (KD) of m2B7 for IL-5Ra was 40.2 nM, and the affinity of hu2B7 was 47.8 nM. The benralizumab analogue had an affinity of 26.8 nM. Therefore, it was confirmed that the order of affinity was benralizumab > m2B7 > hu2B7.

[Table 5]

| | $K_D$(nM) | $k_{on}$ (1/Ms) | $k_{off}$(1/s) |
|---|---|---|---|
| Benralizumab analogue | 26.8±0.52 | (4.11±0.07)×10$^4$ | (1.10±0.01)×10$^{-3}$ |
| m2B7 | 40.2±0.84 | (2.07±0.04)×10$^4$ | (8.34±0.09)×10$^{-4}$ |
| hu2B7 | 47.8±1.06 | (2.22±0.04)×10$^4$ | (1.06±0.01)×10$^{-3}$ |

Example 8: Comparison in inhibition of IL-5-dependent proliferation in cell lines between anti-IL-5Rα antibodies (m2B7, hu2B7, benralizumab analogues)

TF-1 cells stably expressing IL-5Rα (TF-1/IL-5Rα cells) were seeded at 100 μl of $2 \times 10^4$ cells in a 96-well plate. On the same day, 50 μl of 320 pM IL-5 and 50 μl of anti-IL-5Rα mouse antibody solution previously diluted to 80 nM, 400 nM, or 2,000 nM were added and incubated in a 96-well plate at 37°C and 5% $CO_2$ for 40 hours. In order to measure the proliferation capacity of cells, 100 μl of cell suspension was collected from each well, 100 μl of CellTiter-Glo® (manufactured by Promega) was added thereto, reacted at room temperature for 20 minutes and analyzed with a Cytation™ 3 cell imaging multimode reader (FIG. 3B).

The result of the analysis showed that the IL-5 signal blocking effect of hu2B7 was lower than that of m2B7. Similar to the order of high affinity, the IL-5 inhibitory ability was high in the order of benralizumab analogue > m2B7 > hu2B7. Therefore, the present inventors tried to increase the affinity for IL-5Rα based on hu2B7 in order to increase the biological efficacy of the anti-IL-5Rα antibody.

Example 9: Construction of yeast cell surface expression library to increase hu2B7-based affinity

First, in order to express a single-chain Fab (scFab) on the yeast surface, a hu2B7 antibody was constructed in the form of scFab in a pYDS-H vector treated with a NheI/ApaI restriction enzyme to clone the pYDS hu2B7 scFab vector and a pYDS-dummy vector in which a stop codon is generated due to an open reading frame (ORF) shifted due to one additionally introduced nucleotide. By using the pYDS-dummy vector, even if a vector that has not been treated with a restriction enzyme is mixed, only the vector containing the desired library gene can express scFab on the yeast surface.

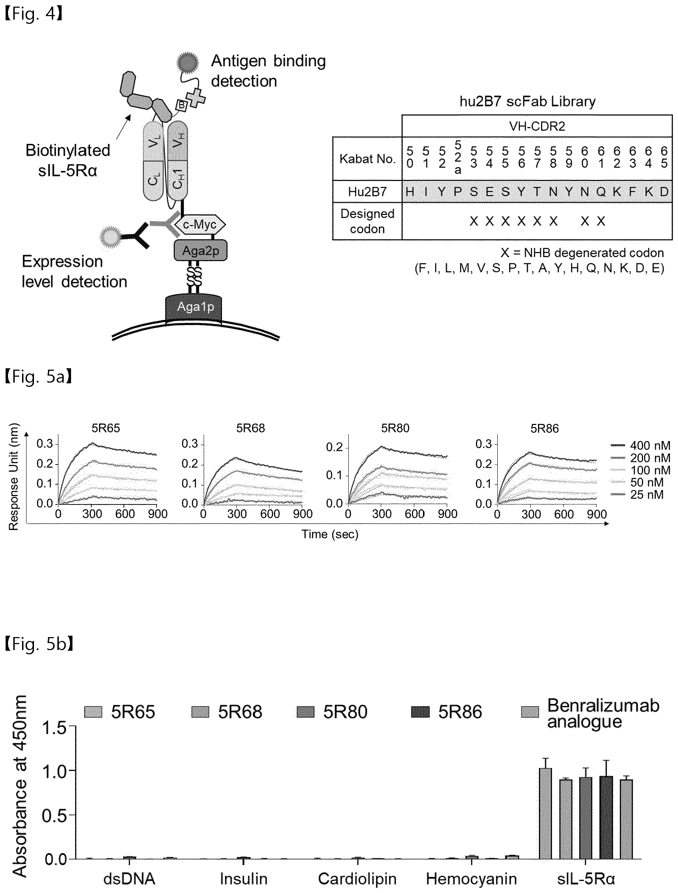

VH-CDR2 (residues 53-58 and 60-61 (Kabat numbering)) was diversified using the NHB degenerate codons (encoding Ala, Asp, Glu, Phe, His, Ile, Lys, Leu, Met, Asn, Pro, Gln, Ser, Thr, Val, and Tyr). The NHB degenerate codons were used because they did not encode Arg, which is known to greatly contribute to antibodies having nonspecificity (FIG. 4).

Overlapping PCR was performed to prepare 12 μg of the library gene and 4 μg of the pYDS dummy vector treated with NheI/ApaI restriction enzymes. Although the pYDS dummy vector that has not been treated with the restriction enzyme remains and thus is transformed into a yeast strain, it is not expressed on the yeast surface by the stop codon. The two genes were mixed and transformed into a yeast AWT101 (MATa, Trp-) strain for yeast surface expression by electroporation, and constructed through homologous recombination. The AWY101 strain is a strain constructed to increase protein expression efficiency on the yeast surface by overexpressing protein disulfide isomerase. This process was repeated 12 times, serial dilution was then performed, and then the library size was detected by measuring the number of colonies grown in a SD-CAA selection medium (20 g/L glucose, 1.7 g/L yeast nitrogen base without amino acids and ammonium sulfate, 5 g/L ammonium sulfate, 5.4 g/L Na $2HPO_4$, 8.6 g/L $NaH_2PO_4$. 5 g/L casamino acids). The library that was produced had a size of about $4\times10^7$.

Example 10: Screening for IL-5Ra antigen protein of yeast scFab library and derivation of clones

In order to select clones with higher affinity, the IL-5Ra antigen protein prepared in <Example 1> was performed according to the manufacturer's protocol using the BirA biotin-protein ligase standard reaction kit (AVIDITU, BIRA500) to prepare biotinylated sIL-5Rα. Yeast clones that specifically bind to biotinylated sIL-5Rα were screened from the constructed library.

Specifically, in order to detect the yeast expression levels of biotinylated sIL-5Rα and scFab, 9E10, a mouse antibody that binds to c-Myc, was prepared at a ratio of 1:200 in a volume of 1 ml. This mixture was bound to yeast cell-expressed scFab ($1\times10^8$ scFab yeast) at room temperature for 30 minutes, and unbound antibodies were washed. PE-conjugated streptavidin (Streptavidin-R-phycoerythrin conjugate (SA-PE), Thermo) was bound to Alexa 488-conjugated anti-mouse IgG antibody (goat Alexa 488-conjugated anti-mouse IgG antibody, Thermo) at 14°C for 10 minutes, and clones with high scFab expression and high binding affinity to biotinylated sIL-5Rα were screened using FACS (fluorescence-activated cell sorting).

While lowering the concentration of the biotinylated sIL-5Rα, clones with high Fab expression and high binding affinity to biotinylated sIL-5Rα were screened from the library using the FACS process (0.5 μM in round 1, 50 nM in round 2, and 10 nM in round 3 and 4), and the derived pool was analyzed. A series of this process was repeated 4 times.

Finally, DNA was obtained from yeast cells expressing each individual clone that specifically binds to sIL-5Rα and was sequenced to select four antibodies, unique base and amino acid sequences of which were identified.

Tables 6 and 7 each show sequences of heavy-chain CDRs and heavy-chain variable regions of four individual clones having the ability to bind to selected IL-5Rα.

[Table 6]

| Heavy-chain variable region name | CDR1 sequence | CDR2 sequence | | | | | | | | | | | | | | | CDR3 sequence | | | | | | | | | | | |
|---|---|---|---|---|---|---|---|---|---|---|---|---|---|---|---|---|---|---|---|---|---|---|---|---|---|---|---|---|
| Kabat No. | 31 32 33 34 35 | 50 51 52 52a 53 54 55 56 57 58 59 60 61 62 63 64 65 | | | | | | | | | | | | | | | 95 96 97 98 99 100 100a 100b 100c 100d 100e 101 102 | | | | | | | | | | | | |
| 5R65 | S Y W I N | H I Y P N K N E N Y Y N H K F K D | | | | | | | | | | | | | | | D Y Y G R S Y Y Y A M D Y | | | | | | | | | | | | |
| | SEQ ID No: 3 | SEQ ID No: 15 | | | | | | | | | | | | | | | SEQ ID No: 5 | | | | | | | | | | | | |
| 5R68 | S Y W I N | H I Y P T A T I A V Y N D K F K D | | | | | | | | | | | | | | | D Y Y G R S Y Y Y A M D Y | | | | | | | | | | | | |
| | SEQ ID No: 3 | SEQ ID No: 16 | | | | | | | | | | | | | | | SEQ ID No: 5 | | | | | | | | | | | | |
| 5R80 | S Y W I N | H I Y P Q K T L T I Y N H K F K D | | | | | | | | | | | | | | | D Y Y G R S Y Y Y A M D Y | | | | | | | | | | | | |
| | SEQ ID No: 3 | SEQ ID No: 17 | | | | | | | | | | | | | | | SEQ ID No: 5 | | | | | | | | | | | | |
| 5R86 | S Y W I N | H I Y P T S S V K F Y N N K F K D | | | | | | | | | | | | | | | D Y Y G R S Y Y Y A M D Y | | | | | | | | | | | | |
| | SEQ ID No: 3 | SEQ ID No: 18 | | | | | | | | | | | | | | | SEQ ID No: 5 | | | | | | | | | | | | |

[Table 7]

| Heavy-chain variable region name | sequence | SEQ ID NO: |
|---|---|---|
| 5R65 | QVQLVQSGAEVKKPGSSVKVSCKASGYTFTSYWINWVRQAPGQGLEWIGHIYPNKNENYYNHKFKDKATLTVDK STNTAYMELSSLRSEDTAVYYCTRDYYGRSYYYAMDYWGQGTTLTVSS | SEQ ID No: 11 |
| 5R68 | QVQLVQSGAEVKKPGSSVKVSCKASGYTFTSYWINWVRQAPGQGLEWIGHIYPTATIAVYNDKFKDKATLTVDKS TNTAYMELSSLRSEDTAVYYCTRDYYGRSYYYAMDYWGQGTTLTVSS | SEQ ID No: 12 |
| 5R80 | QVQLVQSGAEVKKPGSSVKVSCKASGYTFTSYWINWVRQAPGQGLEWIGHIYPQKTLTIYNHKFKDKATLTVDKS TNTAYMELSSLRSEDTAVYYCTRDYYGRSYYYAMDYWGQGTTLTVSS | SEQ ID No: 13 |
| 5R86 | QVQLVQSGAEVKKPGSSVKVSCKASGYTFTSYWINWVRQAPGQGLEWIGHIYPTSSVKFYNNKFKDKATLTVDK STNTAYMELSSLRSEDTAVYYCTRDYYGRSYYYAMDYWGQGTTLTVSS | SEQ ID No: 14 |

Example 11: IgG conversion and identification of selected anti-IL-5Rα antibodies

The 4 antibodies selected in the form of scFab were converted into the form of IgG1, which is a commonly used antibody. The variable regions (VH, VL) of the selected antibodies were introduced into a pcDNA3.4 vector encoding the light-chains (CH1, CH2, CH3) and heavy-chain (CL) of IgG1. The light- and heavy-chains of respective antibodies were cotransformed at a ratio of 1:1 into HEK293F cells such that the light- and heavy- chains were expressed together in the cells.

Proteins were expressed and purified using transient transfection. In a shake flask, HEK293F cells suspensiongrown in serum-free Freestyle 293 expression medium were transfected with a mixture of plasmid and polyethyleneimine (PEI) . Upon 100 mL transfection into the shake flask, HEK293F cells were seeded in 90 ml of medium at a density of $1.0 \times 10^6$ cells/ml and incubated at 120 rpm, 8% $CO_2$, and 37°C. The plasmid was diluted to 125 μg in 5 ml Freestyle 293 expression medium and filtered, and PEI 375 μg (7.5 ug/ml) was mixed with 5 ml of diluted medium and allowed to react at room temperature for 10 minutes. Then, the reacted mixed medium was added to the cells seeded in 90 ml and incubated at 120 rpm and 8% $CO_2$ for 6 days. Proteins were purified from the cell incubation supernatant, which was collected with reference to standard protocols. The antibody was applied to a Protein A Sepharose column and washed with PBS (pH 7.4). The antibody was eluted at pH 3.0 using 0.1 M glycine and 0.5 M NaCl buffer, and the sample was immediately neutralized using 1 M Tris buffer. The eluted protein was concentrated using a Vivaspin 30,000 MWCO (Sartorius) centrifugal concentrator after exchanging the buffer with a storage buffer (PBS, pH 6.5). The absorbance of the purified protein at a wavelength of 562 nm was measured and the amount thereof was quantified using a solution in the BCA protein assay kit (Thermo) according to the drawn standard curve.

Example 12: Analysis of binding ability of selected anti-IL-5Rα humanized antibodies

In order to more quantitatively analyze binding ability to IL-5Ra of anti-IL-5Rα antibodies (5R65, 5R68, 5R80, 5R86), the binding ability was measured according to the protocol suggested by the manufacturer using an Octet QK$^e$ (ForteBio, USA) system. Specifically, 1x kinetic buffer was prepared by diluting 10x kinetic buffer (ForteBio, 18-1105) in PBS. The antibody was diluted to a concentration of 1 ug/ml in 1x kinetic buffer, the IL-5Ra purified in Example 1 was sequentially diluted at concentrations of 400, 200, 100, 12.5, 6.25 and 0 nM in 1x kinetic buffer, and 200 μl of each dilution was injected into an opaque 96-well plate through which light does not pass. Antigen affinity of antibodies was analyzed through the variation in refractive index occurring when the antibody bound to the antigen was detached therefrom while the AHC (anti-human IgG Fc capture) sensor chip moved in the order of 1x kinetic buffer, antibody dilution solution, 1x kinetic buffer, antigen dilution solution, and 1x kinetic buffer. Affinity was calculated with Octet Data Analysis software 11.0 in a 1:1 binding model. The results are shown in FIG. 5A.

Table 8 shows the results of analysis of affinity of selected anti-IL-5Rα antibodies to IL-5Ra using the Octet QK$^e$ system. It was confirmed that the selected clones had an affinity of 11.8 to 24.1 nM which was higher than the affinity (26.8 nM) of the benralizumab analogue.

[Table 8]

|  | $K_D$(nM) | $k_{on}$ (1/Ms) | $k_{off}$(1/s) |
|---|---|---|---|
| 5R65 | 14.5±0.29 | (2.27±0.03)×10⁴ | (3.29±0.05)×10⁻⁴ |
| 5R68 | 24.1±0.47 | (2.35±0.03)×10⁴ | (5.66±0.07)×10⁻⁴ |
| 5R80 | 16.2±0.69 | (2.25±0.05)×10⁴ | (3.65±0.10)×10⁻⁴ |
| SR86 | 11.8±0.44 | (2.76±0.06)×10⁴ | (3.25±0.10)×10⁻⁴ |

Example 13: Analysis of binding specificity of selected anti-IL-5Rα humanized antibodies

The binding specificity of the antibody was determined using indirect ELISA. Multi-antigen ELISA was performed using four structurally different antigenic double-stranded DNA (dsDNA), insulin, the macromolecule hemocyanin, and the membrane component, cardiolipin.

Specifically, IL-5Ra antigen protein (50 ng/well), dsDNA (25 ng/well), insulin (125 ng/well), hemocyanin (125 ng/well), and cardiolipin (1250 ng/well) proteins were immobilized on a 96-well plate at room temperature for 1 hour, and blocked using 2% skim milk containing 0.1% PBST (0.1% Tween20, pH 7.4, 137 mM NaCl, 10 mM phosphate, 2.7 mM KCl) at room temperature for 1 hour. After discarding the solution and then washing the same three times with 0.1% PBST, the antibody was diluted to 100 nM, added to the blocked plate in an amount of 25 μl/well, and allowed to react at room temperature for 1 hour. After discarding the solution and washing the same three times with 0.1% PBST, an anti-human IgG-HRP antibody (1:4000) as a secondary antibody was added in an amount of 25 μl/well and reacted at room temperature for 1 hour. After discarding the solution and washing three times with PBST, a TMB solution was added in an amount of 25 μl/well, color development was induced at room temperature for 2 minutes, the reaction was stopped using $H_2SO_4$ (2N), and the absorbance at 450 nm was measured with an ELISA reader.

As can be seen from the results of FIG. 5B, anti-IL-5Rα humanized antibodies exhibited binding ability only to IL-5Rα, but did not exhibit binding ability to four other types of antigens, which indicates that anti-IL-5Rα humanized antibodies had binding specificity to IL-5Rα.

Example 14: Evaluation of competitive binding to IL-5Rα of selected anti-IL-5Rα humanized antibodies and IL-5

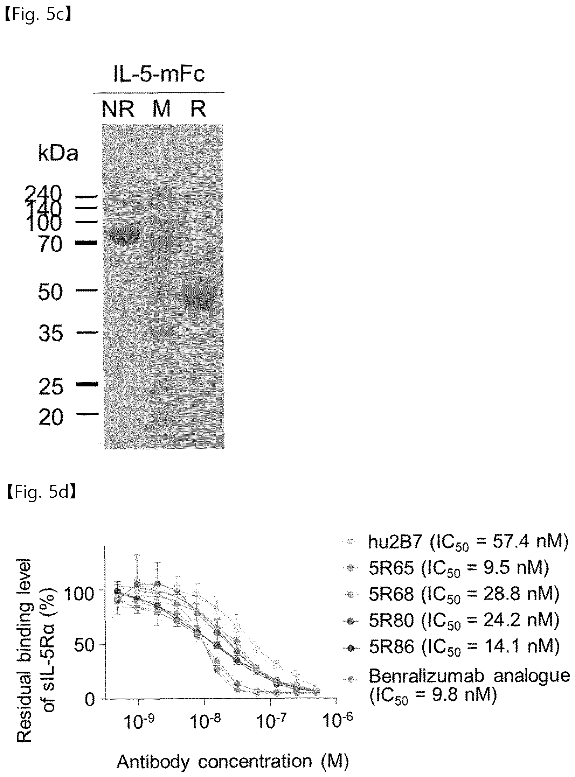

Prior to biological efficacy evaluation, binding competition ELISA was performed to determine whether or not anti-IL-5Rα humanized antibodies bind competitively with IL-5 to the IL-5 binding site of IL-5Rα. Specifically, IL-5 (Uniprot code: P05113, Ile20-Ser134) was fused to the G4S linker and the heavy-chain constant region (hinge-CH2-CH3) of mouse IgG2a, was subjected to transient transfection and purified (FIG. 5C). The purified IL-5-mFc proteins were immobilized at a density of 100 ng/well on a 96-well plate at room temperature for 1 hour, and blocked using 0.1% PBST containing 2% skim milk at room temperature for 1 hour. After discarding the solution and then washing the same three times with 0.1% PBST, mixtures of anti-IL-5α antibody [2-fold dilution at 2,000 nM] at various concentrations were prepared and each mixture was added in an amount of 25 μl/well to the blocked plate, 100 nM IL-5Rα was further added

at 25 $\mu$l/well for 1 hour, followed by reaction at room temperature for 1 hour. The solution was discarded, the residue was washed three times with 0.1% PBST, and an anti-his-HRP antibody (1:2000) was added as a secondary antibody in an amount of 50 $\mu$l/well, followed by reaction at room temperature for 1 hour. After discarding the solution and washing three times with PBST, a TMB solution was added in an amount of 50 $\mu$l/well, color development was induced at room temperature for 2 minutes and 30 seconds, the reaction was stopped using $H_2SO_4$ (2N), and the absorbance at 450 nm was measured. The result showed that anti-IL-5R$\alpha$ humanized antibodies and IL-5-mFc compete for binding to IL-5R$\alpha$ and thereamong, the 5R65 and 5R86 antibodies had $IC_{50}$ of 9.5 nM and 14.1 nM, respectively, which are similar to the $IC_{50}$ (9.8 nM) of the benralizumab analogue (FIG. 5D).

Example 15: Comparison of IL-5-dependent proliferation inhibition in cell lines between selected anti-IL-5R$\alpha$ humanized antibodies

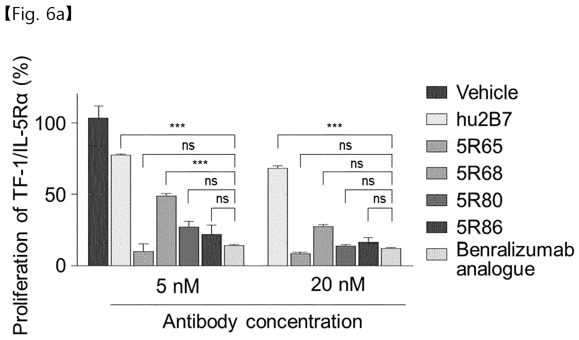

Transformed TF-1 cells stably expressing IL-5R$\alpha$ (TF-1/IL-5R$\alpha$ cells) were seeded at 100 $\mu$l of $2 \times 10^4$ cells in a 96-well plate. On the same day, 50 ul of 640 pM IL-5 and 50 $\mu$l of anti-IL-5R$\alpha$ mouse antibody solution previously diluted to 20 nM and 100 nM were added and incubated in a 96-well plate at 37°C and 5% $CO_2$ for 40 hours. In order to measure the proliferation capacity of cells, 100 $\mu$l of cell suspension was collected from each well, 100 $\mu$l of CellTiter-Glo® (manufactured by Promega) was added thereto, reacted at room temperature for 20 minutes and analyzed with a Cytation™ 3 cell imaging multi-mode reader (FIG. 6A).

The result of the analysis showed that, similar to the result of analysis of $IC_{50}$ of competitive ELISA, the 5R65 antibody exhibited the best IL-5 signal blocking effect. The ability to inhibit bioactivity of the 5R65 and 5R86 antibodies using eosinophils, the cells on which IL-5 acts most in the human body was evaluated.

Example 15: Evaluation of human eosinophil proliferation inhibition of selected anti-IL-5R$\alpha$ humanized antibodies

Eosinophils exhibit activities such as differentiation, proliferation, and migration by IL-5. Since the number of eosinophils in blood is increased in asthmatic patients, the ability of anti-IL-5R$\alpha$ humanized antibodies to inhibit eosinophil proliferation was evaluated to determine whether or not they could contribute to a decrease in the number of eosinophils.

Specifically, 50 ml of Ficoll-Paque solution (GE Healthcare, 17-5442-03) was dispensed into each 50 ml polypropylene centrifuge tube, and 20 ml of the heparinized patient blood was layered on each tube. The result was centrifuged at 879 $\times$ g at room temperature for 25 minutes to separate and collect the lowest layer. 2% dextran solution was dispensed to separate the red blood cells (lower layer) and the granulocyte layer (upper layer) from each other, and the granulocyte layer was recovered and 27 ml of sterilized water and 3 ml of 10$\times$HBSS were added thereto to remove red blood cells mixed therewith. Concentrated granulocytes (eosinophils and neutrophils) from which red blood cells were removed were separated and collected by centrifugation at 300$\times$g and 4°C for 10 minutes. Finally, only eosinophils were purified from granulocytes using a commercially available eosinophil cell isolation kit (Miltenyi Biotec; 130-092-010) according to the manufacturer's protocol.

Eosinophils were seeded at $5\times10^4$ cells/well into a 96-well cell culture plate, human IL-5 having a final concentration of 100 pM was added thereto, and anti-IL-5Ra antibodies having a final concentration of 0.5 $\mu$M, and 1 $\mu$M were each added thereto. Each antibody was cultured in 2 to 3 wells and the capacity of each well was 200 ul. After culturing for 2 days at 37°C in a $CO_2$ incubator, 100 $\mu$l of cell suspension was recovered from each well, and 100 $\mu$l of CellTiter-Glo® Promega sample was added to the culture medium and incubated at room temperature for 20 minutes. The proliferative ability of eosinophils was analyzed by measuring luminescence with a microplate meter.

The antibody used as an isotype control was obtained by purifying an Avastin analogue having an IgG1 constant region prepared by introducing the heavy-chain variable region and light-chain variable region (DrugBank Accession No. DB00112) of Avastin, into a vector pcDNA3.4 encoding heavy-chains (CH1, CH2, CH3) and light-chain (CL) of IgG1, which is a commonly used antibody, as the method designed in <Example 3> by the present inventors.

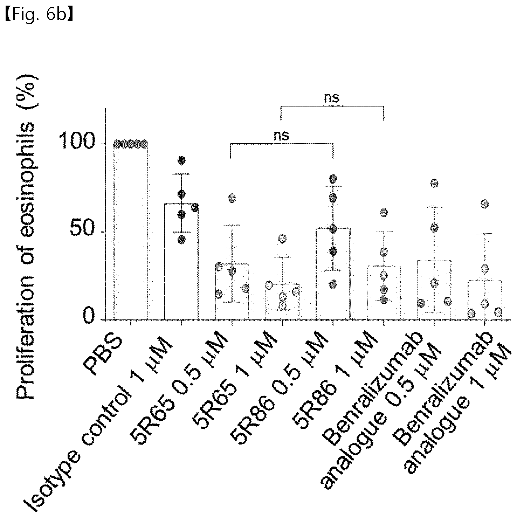

As can be seen from FIG. 6B, the 5R65 antibody inhibited the proliferation of eosinophils to a level similar to that of the benralizumab analogue. On the other hand, 5R86 exhibited lower eosinophil proliferation inhibition than the benralizumab analogue, although there was no significance therebetween. Although 5R65 and 5R86 have higher affinity than the benralizumab analogue, they exhibited similar or lower IL-5 bioactivity inhibition in TF-1/IL-5R$\alpha$ cell lines and eosinophils. An important factor in the antagonistic activity of an antibody is an epitope in addition to affinity. Since IL-5 binds to IL-5Ra such that it covers the entire extracellular domain of IL-5R$\alpha$, domain mapping was performed to determine the domain to which the antibody binds.

Example 16: Domain mapping of 5R65 antibody

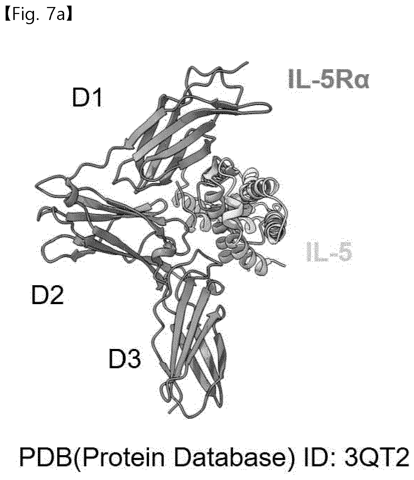

The binding structure of IL-5 and IL-5Ra is shown in PDB (protein database) ID: 3QT2, and the extracellular domain of IL-5Ra consists of three domains and is bent like a wrench, such that IL-5 is in contact with all three domains (FIG. 7A). Domain 1 (D1) represented by SEQ ID NO: 19 in IL-5Ra represents Gly103 in ASp1, domain 2 (D2) represents Asn220 in Ser104, and domain 3 (D3) represents Trp322 in Pro221. Thereamong, domain 1 (D1), a membrane-distal domain, contributes the most to IL-5 binding. The benralizumab analogue has an epitope on D1 of IL-5Ra (Kolbeck et al., 2010).

[Table 9]

SEQ ID No: 19

```
1              5              10             15             20
Asp Leu Leu Pro Asp Glu Lys Ile Ser Leu Leu Pro Pro Val Asn Phe Thr Ile Lys Val
21             25             30             35             40
Thr Gly Leu Ala Gln Val Leu Leu Gln Trp Lys Pro Asn Pro Asp Gln Glu Gln Arg Asn
41             45             50             55             60
Val Asn Leu Glu Tyr Gln Val Lys Ile Asn Ala Pro Lys Glu Asp Asp Tyr Glu Thr Arg
61             65             70             75             80
Ile Thr Glu Ser Lys Cys Val Thr Ile Leu His Lys Gly Phe Ser Ala Ser Val Arg Thr
81             85             90             95             100
Ile Leu Gln Asn Asp His Ser Leu Leu Ala Ser Ser Trp Ala Ser Ala Glu Leu His Ala
101            105            110            115            120
Pro Pro Gly Ser Pro Gly Thr Ser Ile Val Asn Leu Thr Cys Thr Thr Asn Thr Thr Glu
121            125            130            135            140
Asp Asn Tyr Ser Arg Leu Arg Ser Tyr Gln Val Ser Leu His Cys Thr Trp Leu Val Gly
141            145            150            155            160
Thr Asp Ala Pro Glu Asp Thr Gln Tyr Phe Leu Tyr Tyr Arg Tyr Gly Ser Trp Thr Glu
161            165            170            175            180
Glu Cys Gln Glu Tyr Ser Lys Asp Thr Leu Gly Arg Asn Ile Ala Cys Trp Phe Pro Arg
181            185            190            195            200
Thr Phe Ile Leu Ser Lys Gly Arg Asp Trp Leu Ala Val Leu Val Asn Gly Ser Ser Lys
201            205            210            215            220
His Ser Ala Ile Arg Pro Phe Asp Gln Leu Phe Ala Leu His Ala Ile Asp Gln Ile Asn
221            225            230            235            240
Pro Pro Leu Asn Val Thr Ala Glu Ile Glu Gly Thr Arg Leu Ser Ile Gln Trp Glu Lys
241            245            250            251            260
Pro Val Ser Ala Phe Pro Ile His Cys Phe Asp Tyr Glu Val Lys Ile His Asn Thr Arg
261            285            270            275            280
Asn Gly Tyr Leu Gln Ile Glu Lys Leu Met Thr Asn Ala Phe Ile Ser Ile Ile Asp Asp
281            285            290            295            300
Leu Ser Lys Tyr Asp Val Gln Val Arg Ala Ala Val Ser Ser Met Cys Arg Glu Ala Gly
301            305            310            315            320
Leu Trp Ser Glu Trp Ser Gln Pro Ile Tyr Val Gly Asn Asp Glu His Lys Pro Leu Arg
322
Glu Trp
```

Based on the fact that benralizumab does not bind to mouse IL-5Ra (mouse IL-5Rα, mIL-5Rα), domain mapping was performed using variant IL-5Rα constructed by combining the domain of mouse IL-5Rα with the domain of human IL-5Rα (human IL-5Rα, IL-5Rα). Based thereon, the present inventors also expressed the three modified IL-5Rα obtained by substituting the mouse IL-5Rα domain with each domain corresponding to the human IL-5Rα domain on the surface of yeast. Domain 1 (D1) represented by SEQ ID NO: 20 in mIL-5Rα represents Gly103 in ASp1, domain 2 (D2) represents Asn220 in Ser104, and domain 3 (domain 3, D3) represents Trp321 in Pro221. FIG. 7B is a schematic diagram illustrating three types of human IL-5Rα, mouse IL-5Rα, and human-mouse variant IL-5Rα.

[Table 10]

SEQ ID No: 20

```
1              5                    10                   15                   20
Asp Leu Leu Asn His Lys Lys Phe Leu Leu Leu Pro Pro Val Asn Phe Thr Ile Lys Ala
21             25                   30                   35                   40
Thr Gly Leu Ala Gln Val Leu Leu His Trp Asp Pro Asn Pro Asp Gln Glu Gln Arg His
41             45                   50                   55                   60
Val Asp Leu Glu Tyr His Val Lys Ile Asn Ala Pro Gln Glu Asp Glu Tyr Asp Thr Arg
61             65                   70                   75                   80
Lys Thr Glu Ser Lys Cys Val Thr Pro Leu His Glu Gly Phe Ala Ala Ser Val Arg Thr
81             85                   90                   95                   100
Ile Leu Lys Ser Ser His Thr Thr Leu Ala Ser Ser Trp Val Ser Ala Glu Leu Lys Ala
101            105                  110                  115                  120
Pro Pro Gly Ser Pro Gly Thr Ser Val Thr Asn Leu Thr Cys Thr Thr His Thr Val Val
121            125                  130                  135                  140
Ser Ser His Thr His Leu Arg Pro Tyr Gln Val Ser Leu Arg Cys Thr Trp Leu Val Gly
141            145                  150                  155                  160
Lys Asp Ala Pro Glu Asp Thr Gln Tyr Phe Leu Tyr Tyr Arg Phe Gly Val Leu Thr Glu
161            165                  170                  175                  180
Lys Cys Gln Glu Tyr Ser Arg Asp Ala Leu Asn Arg Asn Thr Ala Cys Trp Phe Pro Arg
181            185                  190                  195                  200
Thr Phe Ile Asn Ser Lys Gly Phe Glu Gln Leu Ala Val His Ile Asn Gly Ser Ser Lys
201            205                  210                  215                  220
Arg Ala Ala Ile Lys Pro Phe Asp Gln Leu Phe Ser Pro Leu Ala Ile Asp Gln Val Asn
221            225                  230                  235                  240
Pro Pro Arg Asn Val Thr Val Glu Ile Glu Ser Asn Ser Leu Tyr Ile Gln Trp Glu Lys
241            245                  250                  255                  260
Pro Leu Ser Ala Phe Pro Asp His Cys Phe Asn Tyr Glu Leu Lys Ile Tyr Asn Thr Lys
261            265                  270                  275                  280
Asn Gly His Ile Gln Lys Glu Lys Leu Ile Ala Asn Lys Phe Ile Ser Lys Ile Asp Asp
281            285                  290                  295                  300
Val Ser Thr Tyr Ser Ile Gln Val Arg Ala Ala Val Ser Ser Pro Cys Arg Met Pro Gly
301            305                  310                  315                  320
Arg Trp Gly Glu Trp Ser Gln Pro Ile Tyr Val Gly Lys Glu Arg Lys Ser Leu Val Glu
321
Trp
```

Specifically, in order to detect the yeast expression level of IL-5Rα, 9E10, a mouse antibody that binds to c-Myc, was bound at a ratio of 1:200 in a volume of 1 ml at room temperature for 30 minutes, and unbound antibodies were washed away. Alexa 488-conjugated anti-mouse IgG antibody (goat Alexa 488-conjugated anti-mouse IgG antibody, Thermo) was bound thereto at 14°C for 10 minutes, and analyzed using a flow cytometer.

In order to determine the binding ability of the antibody, 100 nM of the antibody was combined with $10^7$ yeast cells at room temperature for 30 minutes. Then, a goat Alexa 488-conjugated anti-mouse IgG antibody (Thermo) was bound at 4°C for 15 minutes and then analyzed by flow cytometry.

As a result, as can be seen from FIG. 7C, the benralizumab analogue exhibited binding ability to yeast expressing human IL-5Rα, but did not have binding ability to mouse IL-5Rα. In addition, similar to the previous results, the benralizumab analogue exhibited binding ability to human IL-5Ra containing human IL-5Ra domain 1 (D1) and hD1-mD2-mD3 IL-5Ra modified IL-5Rα. The 5R65 antibody also exhibited binding ability to yeast expressing human IL-5Rα, but did not exhibit binding ability to mouse IL-5Rα. However, unlike benralizumab analogues, the benralizumab analogue exhibited binding ability to human IL-5Ra containing human IL-5Ra domain 3 (D3) and to mD1-mD2-hD3 IL-5Rα-modified IL-5Rα.

This means that 5R65 has a different epitope from the benralizumab analogue. Specifically, 5R65 has an epitope at D3, a membrane-proximal domain, whereas the benralizumab analogue has an epitope at D1, a membrane-distal domain. 5R65 has similar IL-5 bioactivity inhibition to the benralizumab analogue although it has an affinity about twice as high as that of the benralizumab analogue. Then, one more round of affinity maturation was performed based on the assumption that higher affinity is required to provide higher IL-5 biological activity inhibition than the benralizumab analogue.

Example 17: High-diversity antibody library construction and screening for further affinity improvement based on 5R65 antibody

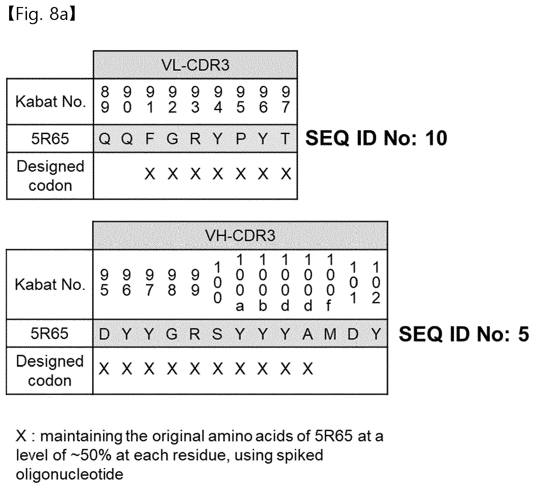

A library for affinity maturation was constructed by simultaneously diversifying VL-CDR3 and VH-CDR3. All residues excluding residues Q89 and Q90 of VL-CDR3 and residues M100f, D101, and Y102 of VH-CDR3, which are generally highly conserved in human germline genes, were diversified. Mutations in the CDRs may result in loss of antigen-binding ability or an epitope different from that of the parent antibody (5R65). To minimize this, a handmixed spiked oligonucleotide encoding a residue of 5R65 with 50% probability was used. A schematic diagram thereof is shown in FIG. 8A.

In order to increase the antagonistic activity of the antibody, it is important to maintain the binding of the antibody to the receptor. For this purpose, an antibody with a low $K_{off}$ rate is required. Therefore, kinetic screening was performed.

Yeast binding to biotinylated sIL-5Rα was initially screened by a first round of magnetic activated cell sorting (MACS). Subsequently, 4 rounds of FACS were screened by kinetic screening.

Specifically, the library was saturated with 5 nM of biotinylated sIL-5Rα, suspended in 1 ml dissociation buffer containing a 2-fold molar excess of non-biotinylated sIL-5Rα and incubated at 37°C to prevent the dissociated biotinylated sIL-5Rα from re-binding to the yeast surface. The dissociation time was increased by 1 hour for each subsequent round. Every hour the dissociation buffer was removed and resuspension in fresh dissociation buffer was performed. After dissociation, 9E10, a mouse antibody that binds to c-Myc, was prepared at a ratio of 1:200 in a volume of 100 μl to detect the yeast expression level of scFab. Then, PE-conjugated streptavidin (Streptavidin-R-phycoerythrin conjugate (SA-PE), Thermo) was bound to Alexa 488-conjugated anti-mouse IgG antibody (goat Alexa 488-conjugated anti-mouse IgG antibody, Thermo) at 4°C for 15 minutes, clones with high scFab expression and high binding affinity to biotinylated sIL-5Rα were selected using sIL-5Rα FACS (fluorescence-activated cell sorting).

As a result, 6 new clones were isolated. Surprisingly, all clones had no mutation in the VL-CDR3 sequence and had a mutation only in the VH-CDR3 sequence. The clones were converted to human IgG1 form and then purified.

Tables 11 and 12 show sequences of heavy-chain CDRs and heavy-chain variable regions of the six selected individual clones.

[Table 11]

| Heavy-chain variable region name | CDR1 sequence | CDR2 sequence | CDR3 sequence |
|---|---|---|---|
| Kabat No. | 31 32 33 34 35 | 50 51 52 52a 53 54 55 56 57 58 59 60 61 62 63 64 65 | 95 96 97 98 99 100 100a 100b 100c 100d 100e 101 102 |
| 5R65.7 | S Y W I N | H I Y P N K N E N Y Y N H K F K D | E F Y G R Q Y Y Q A M D Y |
| | SEQ ID No: 3 | SEQ ID No: 15 | SEQ ID No: 27 |
| 5R65.10 | S Y W I N | H I Y P N K N E N Y Y N H K F K D | E Y Y G R S Y Y A A M D Y |
| | SEQ ID No: 3 | SEQ ID No: 15 | SEQ ID No: 28 |
| 5R65.14 | S Y W I N | H I Y P N K N E N Y Y N H K F K D | E H Y G R P Y Y N A M D Y |
| | SEQ ID No: 3 | SEQ ID No: 15 | SEQ ID No: 29 |
| 5R65.18 | S Y W I N | H I Y P N K N E N Y Y N H K F K D | E Y Y G R T Y Y S A M D Y |
| | SEQ ID No: 3 | SEQ ID No: 15 | SEQ ID No: 30 |
| 5R65.39 | S Y W I N | H I Y P N K N E N Y Y N H K F K D | E F Y G R S R Y S A M D Y |
| | SEQ ID No: 3 | SEQ ID No: 15 | SEQ ID No: 31 |
| 5R65.45 | S Y W I N | H I Y P N K N E N Y Y N H K F K D | E Y Y G R S Y Y N A M D Y |
| | SEQ ID No: 3 | SEQ ID No: 15 | SEQ ID No: 32 |

[Table 12]

| Heavy-chain variable region name | sequence | SEQ ID NO: |
|---|---|---|
| 5R65.7 | QVQLVQSGAEVKKPGSSVKVSCKASGYTFTSYWINWVRQAPGQGLEWIGHIYPNKNENYYNHKFKDKATLTVDKSTNTAYMELSSLRSEDTAVYYCTREFYGRQYYQAMDYWGQGTTLTVSS | SEQ ID No: 21 |
| 5R65.10 | QVQLVQSGAEVKKPGSSVKVSCKASGYTFTSYWINWVRQAPGQGLEWIGHIYPNKNENYYNHKFKDKATLTVDKSTNTAYMELSSLRSEDTAVYYCTREYYGRSYYAAMDYWGQGTTLTVSS | SEQ ID No: 22 |
| 5R65.14 | QVQLVQSGAEVKKPGSSVKVSCKASGYTFTSYWINWVRQAPGQGLEWIGHIYPNKNENYYNHKFKDKATLTVDKSTNTAYMELSSLRSEDTAVYYCTREHYGRPYYNAMDYWGQGTTLTVSS | SEQ ID No: 23 |
| 5R65.18 | QVQLVQSGAEVKKPGSSVKVSCKASGYTFTSYWINWVRQAPGQGLEWIGHIYPNKNENYYNHKFKDKATLTVDKSTNTAYMELSSLRSEDTAVYYCTREYYGRTYYSAMDYWGQGTTLTVSS | SEQ ID No: 24 |

(continued)

| Heavy-chain variable region name | sequence | SEQ ID NO: |
|---|---|---|
| 5R65.39 | QVQLVQSGAEVKKPGSSVKVSCKASGYTFTSYWINWVRQAPGQGLEWIGHIYPNKNENYYNHKFKDKATLTVDK STNTAYMELSSLRSEDTAVYYCTREFYGRSRYSAMDYWGQGTTLTVSS | SEQ ID No: 26 |
| 5R66.45 | QVQLVQSGAEVKKPGSSVKVSCKASGYTFTSYWINWVRQAPGQGLEWIGHIYPNKNENYYNHKFKDKATLTVDK STNTAYMELSSLRSEDTAVYYCTREYYGRSYYNAMDYWGQGTTLTVSS | SEQ ID No: 26 |

Example 18: Analysis of binding ability of selected anti-IL-5Rα humanized antibodies selected through 5R65-based affinity maturation

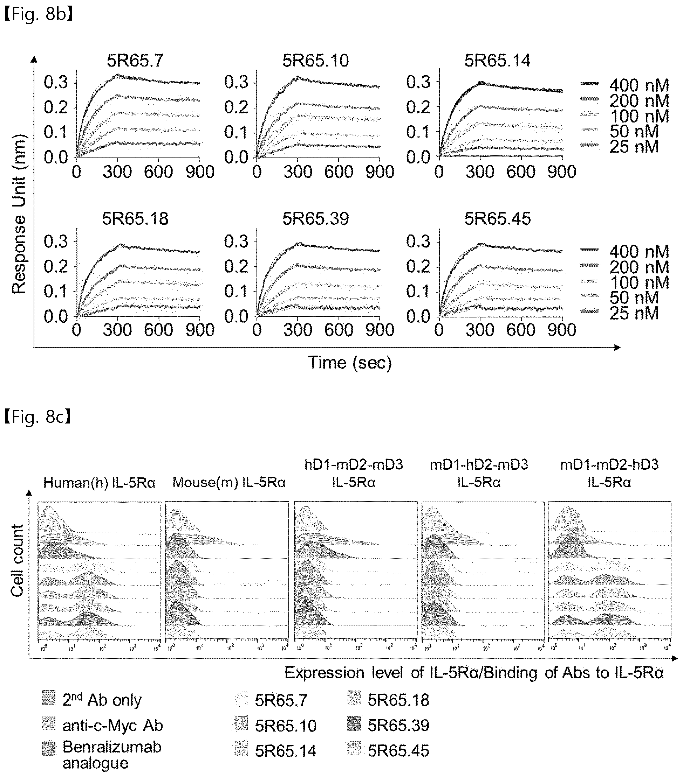

In order to more quantitatively analyze binding ability to IL-5Ra of anti-IL-5Rα antibodies (5R65.7, 5R65.10, 5R65.14, 5R65.18, 5R65.39, 5R65.45), the binding ability was measured according to the protocol suggested by the manufacturer using an Octet QK$^e$ (ForteBio, USA) system. Specifically, 1x kinetic buffer was prepared by diluting 10x kinetic buffer (ForteBio, 18-1105) in PBS. The antibody was diluted to a concentration of 1 ug/ml in 1x kinetic buffer, the IL-5Ra purified in Example 1 was sequentially diluted at concentrations of 400, 200, 100, 12.5, 6.25 and 0 nM in 1x kinetic buffer, and 200 μl of each dilution was injected into an opaque 96-well plate through which light does not pass. Antigen affinity of antibodies was analyzed through the variation in refractive index occurring when the antibody bound to the antigen was detached therefrom while the AHC (anti-human IgG Fc capture) sensor chip moved in the order of 1x kinetic buffer, antibody dilution solution, 1x kinetic buffer, antigen dilution solution, and 1x kinetic buffer. Affinity was calculated with Octet Data Analysis software 11.0 in a 1:1 binding model. The results are shown in FIG. 8B.

Table 13 shows the results of analysis of affinity of selected anti-IL-5Rα antibodies to IL-5Ra using the Octet QK$^e$ system. It was confirmed that all the selected clones had a single digit affinity ($K_D$), i.e., 4.64 to 8.64 nM which was higher than the parent antibody 5R65.

[Table 13]

|  | $K_D$(nM) | $k_{on}$(1/Ms) | $k_{off}$(1/s) |
|---|---|---|---|
| 5R65.7 | $4.64\pm0.23$ | $(3.17\pm0.05)\times10^4$ | $(1.47\pm0.07)\times10^{-4}$ |
| 5R65.10 | $8.25\pm0.40$ | $(2.44\pm0.05)\times10^4$ | $(2.01\pm0.09)\times10^{-4}$ |
| 5R65.14 | $8.64\pm0.35$ | $(2.30\pm0.04)\times10^4$ | $(1.99\pm0.07)\times10^{-4}$ |
| 5R65.18 | $5.95\pm0.27$ | $(2.24\pm0.03)\times10^4$ | $(1.33\pm0.06)\times10^{-4}$ |
| 5R65.39 | $6.13\pm0.32$ | $(2.80\pm0.05)\times10^4$ | $(1.71\pm0.08)\times10^{-4}$ |
| 5R65.45 | $7.26\pm0.36$ | $(2.49\pm0.05)\times10^4$ | $(1.81\pm0.08)\times10^{-4}$ |

Example 19: Domain mapping of anti-IL-5Rα humanized antibodies with improved affinity

Specifically, in order to detect the yeast expression level of IL-5Rα, 9E10, a mouse antibody that binds to c-Myc, was bound at a ratio of 1:200 in a volume of 100 μl at room temperature for 30 minutes, and unbound antibodies were washed. Alexa 488-conjugated anti-mouse IgG antibody (goat Alexa 488-conjugated anti-mouse IgG antibody, Thermo) was further bound at 4°C for 15 minutes, and analyzed using a flow cytometer.

In order to determine the binding ability of the antibody, 100 nM of the antibody was combined with 10$^7$ yeast cells at room temperature for 30 minutes. Then, a goat Alexa 488-conjugated anti-mouse IgG antibody (Thermo) was bound at 4°C for 15 minutes and then analyzed by flow cytometry.

As a result, as can be seen from FIG. 8C, like 5R65, antibodies that had affinity matured based on 5R65 exhibited binding ability to yeast expressing human IL-5Ra and mD1-mD2-hD3 IL-5Rα, and had an epitope in D3, a membrane-proximal domain, which is different from that of the benralizumab analogue, which has an epitope in the membrane-distal domain.

Example 20: Comparison of IL-5-dependent proliferation inhibition in TF-1/IL-5Rα cell lines between anti-IL-5Rα mouse antibodies with improved affinity

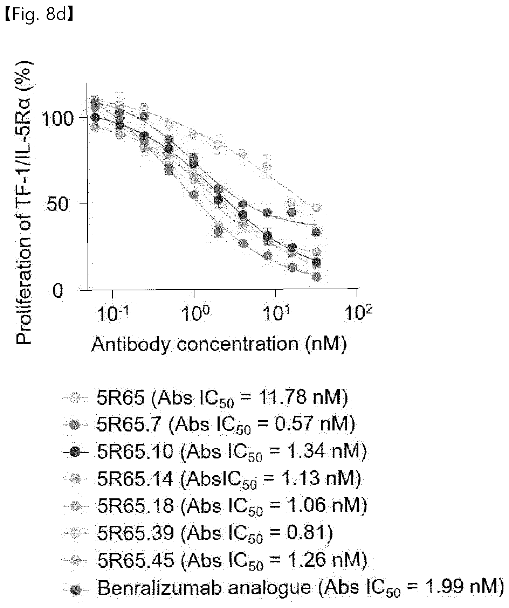

Transformed TF-1 cells stably expressing IL-5Ra (TF-1/IL-5Rα cells) were seeded at 100 µl of $2 \times 10^4$ cells in a 96-well plate. On the same day, 50 µl of 320 pM IL-5 and 50 µl of anti-IL-5Rα mouse antibody solution previously diluted to 128 nM were added and incubated in a 96-well plate at 37°C and 5% $CO_2$ for 40 hours. In order to measure the proliferation capacity of cells, 100 µl of cell suspension was collected from each well, 100 µl of CellTiter-Glo® (manufactured by Promega) was added thereto, reacted at room temperature for 20 minutes and analyzed with a Cytation™ 3 cell imaging multi-mode reader (FIG. 8D). The result showed that the benralizumab analogue had absolute $IC_{50}$ (Abs $IC_{50}$) of -1.99 nM, and the selected anti-IL-5Rα antibodies (5R65.7, 5R65.10, 5R65.14, 5R65.18, 5R65.39, 5R65.45) had abs $IC_{50}$ in the range of 0.57 nM to 1.34 nM, which indicates that the selected anti-IL-5Rα antibodies exhibited improved inhibition of IL-5-dependent proliferation in TF-1/IL-5Rα cell lines compared to the benralizumab analogue. In particular, 5R65.7 exhibited the strongest proliferation inhibition (FIG. 8D).

Example 21: Confirmation of IL-5Ra expression in human granulocytes from healthy donors and severe asthma patients

Prior to evaluating the bioactivity of the anti-IL-5Rα antibodies using eosinophils, the expression of IL-5Ra in eosinophils in the granulocyte layer was observed in peripheral blood from healthy controls.

Specifically, 20 ml of Ficoll-Paque solution (GE Healthcare, 17-5442-03) was dispensed into each 50 ml polypropylene centrifuge tube, and 20 ml of the heparinized patient blood was layered on each tube. The result was centrifuged at $879 \times$ g at room temperature for 25 minutes to separate and collect the lowest layer. 2% dextran solution was dispensed to separate the red blood cells (lower layer) and the granulocyte layer (upper layer) from each other, and the granulocyte layer was recovered and 27 ml of sterilized water and 3 ml of 10×HBSS were added thereto to remove red blood cells mixed therewith. Concentrated granulocytes (eosinophils and neutrophils) from which red blood cells were removed were separated and collected by centrifugation at 300×g and 4°C for 10 minutes.

The enriched granulocytes can be classified into eosinophils and neutrophils depending on the expression of siglec 8 (sialic acid-binding immunoglobulin-like lectin). Eosinophils express siglec-8 and do not express neutrophils. Specifically, granulosa cells concentrated at a density of $1 \times 10^6$/donor sample, 5 µl of APC anti-human Siglec-8 Ab (Biolegen; 347105), and 0.5 µl of PE anti-human IL-5Ra Ab (BD Pharmingen; 555902) were mixed, reacted at 4°C for 30 minutes, washed with PBSM (Miltenyi Biotec; 130-091-221), and then analyzed using a FACS Calibur (BD Bioscience) flow cytometer. After analysis, a dot graph of each sample was obtained.

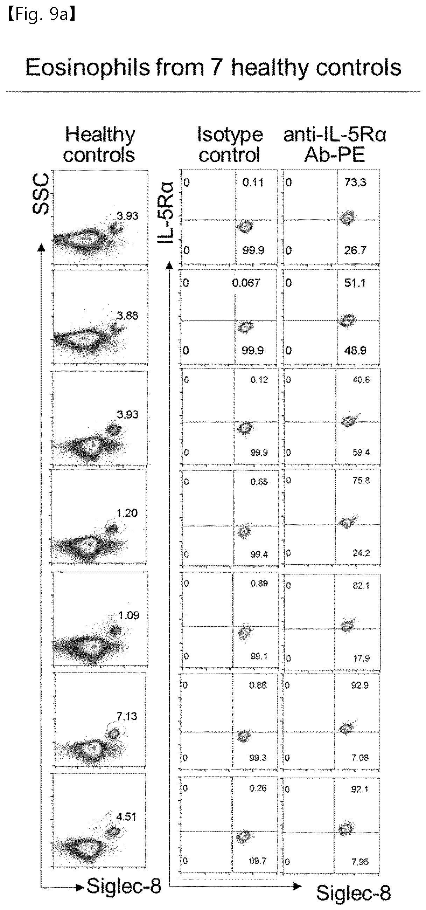

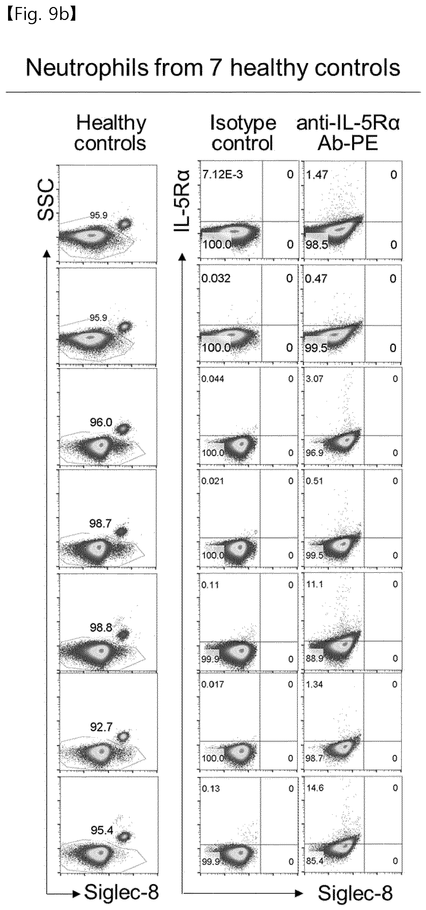

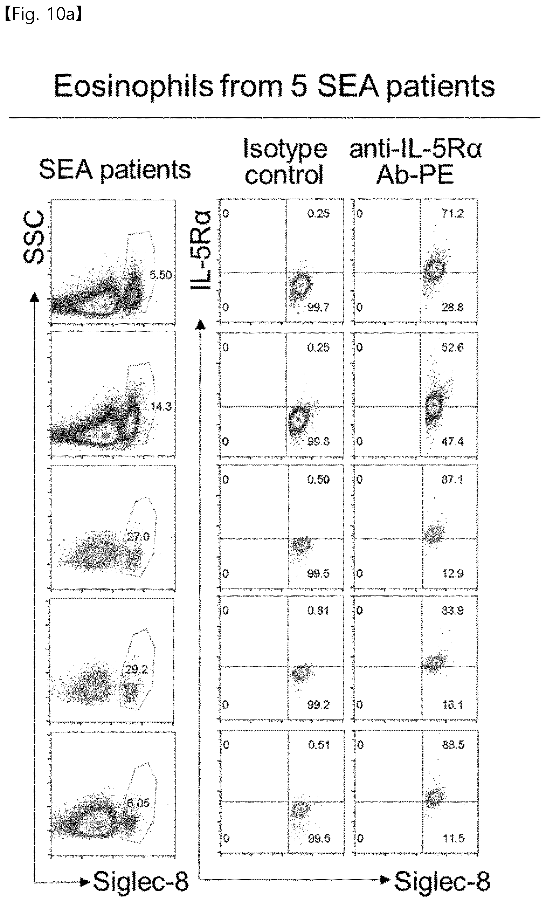

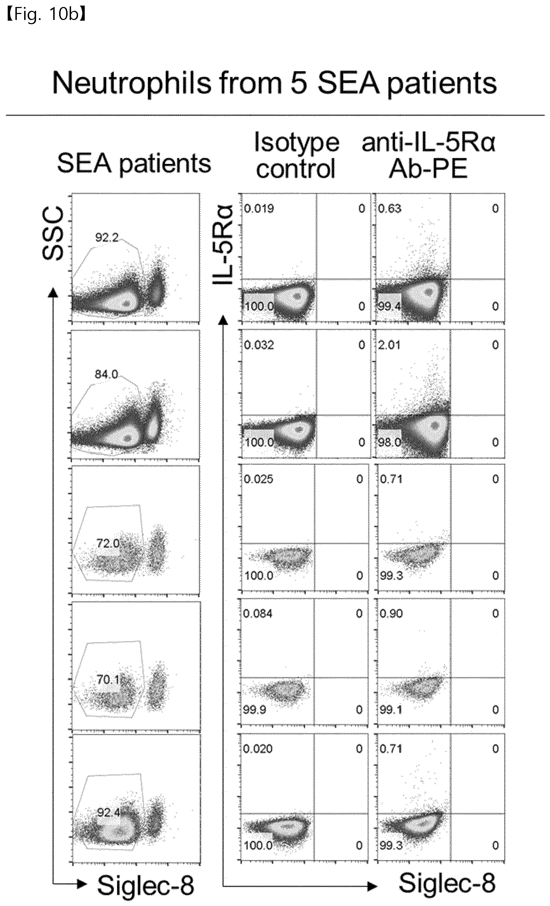

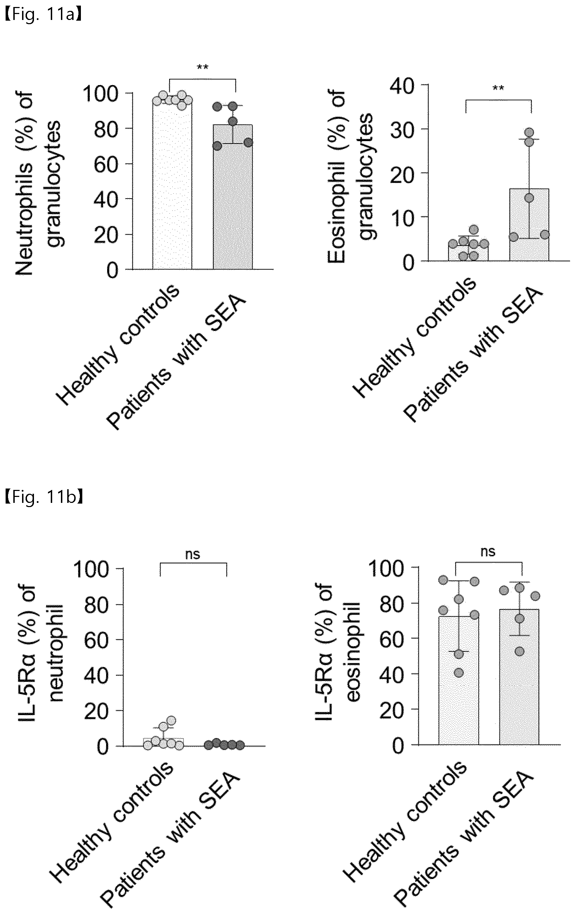

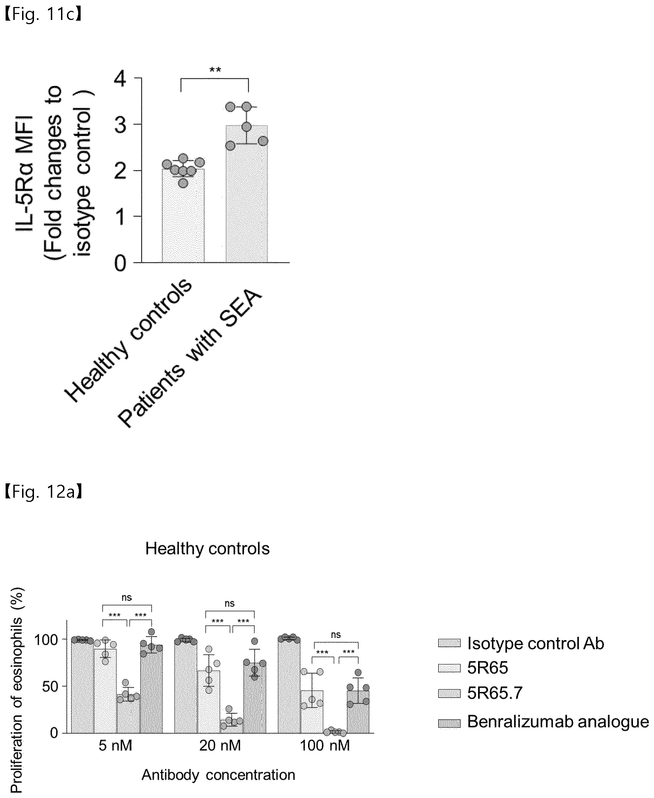

Table 9 shows the gating strategy in which eosinophils were classified depending on the presence or absence of siglec-8 expression in enriched granulocytes derived from healthy donors and the result of confirming the expression of IL-5Ra in eosinophils (FIG. 9A) and neutrophils (FIG. 9B). Table 10 shows the gating strategy in which eosinophils were classified depending on the presence or absence of siglec-8 expression in enriched granulocytes derived from healthy donors and the result of confirming the expression of IL-5Ra in eosinophils (FIG. 10A) and neutrophils (FIG. 10B). FIG. 11A exhibits the ratio of eosinophils and neutrophils in the enriched granulosa cells determined in FIGS. 10 and 9. The patients with severe asthma had significantly higher eosinophils than healthy donors and significantly lower neutrophils than the healthy donors. FIG. 11B shows that neutrophils rarely express IL-5Rα and there is no difference in the ratio of eosinophils expressing IL-5Ra between severe asthmatic patients and healthy donors, but the severe asthmatic patients had a higher expression level (FIG. 11C).

Example 22: Evaluation of the ability of anti-IL-5Rα humanized antibodies with improved affinity to inhibit the proliferation of human eosinophils derived from healthy donors and patients with severe asthma

The inhibition of eosinophil proliferation determined in Example 15 above was determined for 5R65.7 antibody, among the anti-IL-5Rα humanized antibodies with improved affinity.

Specifically, 20 ml of Ficoll-Paque solution (GE Healthcare, 17-5442-03) was dispensed into each 50 ml polypropylene centrifuge tube and 20 ml of the heparinized patient blood was layered on each tube. The result was centrifuged at $879 \times$ g at room temperature for 25 minutes to separate and collect the lowest layer. 2% dextran solution was dispensed to separate the red blood cells (lower layer) and the granulocyte layer (upper layer) from each other, and the granulocyte layer was recovered and 27 ml of sterilized water and 3 ml of 10×HBSS were added thereto to remove red blood cells mixed therewith. Concentrated granulocytes (eosinophils and neutrophils) from which red blood cells were removed were separated and collected by centrifugation at 300×g and 4°C for 10 minutes. Finally, only eosinophils were purified from granulocytes using a commercially available eosinophil cell isolation kit (Miltenyi Biotec; 130-092-010) according to the manufacturer's protocol.

Eosinophils were seeded at $5 \times 10^4$ cells/well into a 96-well cell culture plate, human IL-5 having a final concentration of 100 pM was added thereto, and anti-IL-5Ra antibodies having a final concentration of 5 nM, 20 nM, and 100 nM were each added thereto. Each antibody was cultured in 2 to 3 wells and the capacity of each well was 200 ul. After culturing for 2 days at 37°C in a $CO_2$ incubator, 100 $\mu$l of cell suspension was recovered from each well, and 100 $\mu$l of CellTiter-Glo® Promega sample was added to the culture medium and incubated at room temperature for 20 minutes. The proliferative ability of eosinophils was analyzed by measuring luminescence with a microplate meter.

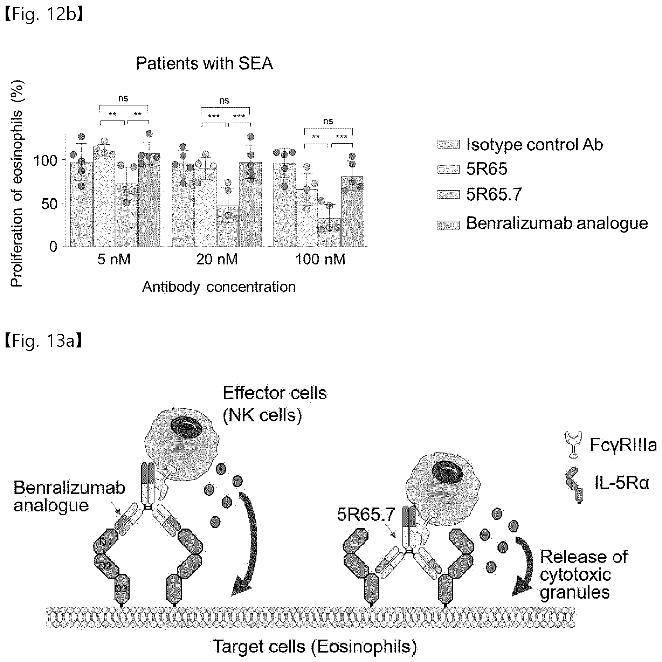

The result of the analysis showed that 5R65.7 exhibited the strongest eosinophil proliferation inhibition which was higher than that of benralizumab analogue (FIG. 12). The effect of the antibodies on eosinophils from severe asthma patients (FIG. 12B) was lower than that of eosinophils from healthy donors (FIG. 12A). The reason for this is that signaling by IL-5 is stronger since the expression of IL-5Ra in eosinophils of patients with severe asthma was higher than that of healthy donors.

Example 23: Evaluation of ability of anti-IL-5R$\alpha$ humanized antibodies to induce antibody-dependent cellular cytotoxicity of human eosinophils from healthy donors and severe asthma patients

Benralizumab has an afucosylated Fc and thus has a higher affinity for Fc$\gamma$RIIIa expressed in NK cells than IgG1 Fc, and exhibits higher ADCC activity. Due to the high ADCC activity thereof, benralizumab can effectively reduce the number of eosinophils in asthmatic patients. Since the present inventors do not have facilities for producing afucosylated antibodies, they compared the activity of benralizumab analogues having an IgG1 Fc with anti-IL-5R$\alpha$ humanized antibodies (5R65, 5R65.7) also having the same IgG1 Fc.

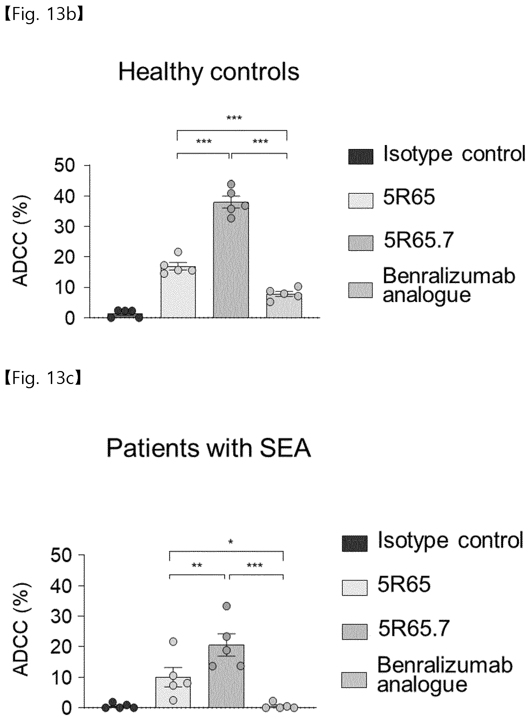

FIG. 13A is a schematic diagram illustrating the antibody-dependent cellular cytotoxicity (ADCC) depending on the binding of the benralizumab analogue and 5R65 and 5R65.7 to IL-5R$\alpha$. The benralizumab analogue binds to D1 of IL-5R$\alpha$, and 5R65 and 5R65.7 bind to D3. Previous studies have reported that, as the distance (immunological synapse) between the effector cells (ex. NK cells) and target cells (ex. eosinophils) increases, the ADCC activity increases. Therefore, 5R65 and 5R65.7 are expected to exhibit higher ADCC activity.

Specifically, 20 ml of Ficoll-Paque solution (GE Healthcare, 17-5442-03) was dispensed into each 50 ml polypropylene centrifuge tube, and 20 ml of heparinized patient blood was layered on each tube. The result was centrifuged at 879 $\times$ g at room temperature for 25 minutes to separate and recover the buffy coat layer for NK cell isolation and the lowermost layer for eosinophil isolation. Isolated primary NK cells and eosinophils were used as effector and target cells at a ratio of 5:1, respectively. Eosinophils and primary NK cells are seeded at densities of $5 \times 10^4$ cells/well and $2.5 \times 10^5$ cells/well, respectively, into a 96-well cell culture plate, and human IL-5 having a final concentration of 100 pM was added thereto. In addition, various anti-IL-5R$\alpha$ antibodies with improved affinities having a final concentration of 1 $\mu$M were added thereto. Each antibody was cultured in two wells and the final volume of each well was adjusted to 200 $\mu$l. The cells were incubated in a $CO_2$ incubator at 37°C for 20 hours, and 2 hours before collection of the cell suspension, 20 $\mu$l of a lysis solution is added to a sample for maximum lactate dehydrogenase (LDH) efflux. After the completion of culture, 50 $\mu$l of the cell suspension was collected from each well, and then 50 ul of CytoTox96® Reagent (manufactured by Promega) was added thereto and the result was incubated at room temperature for 30 minutes. After adding 50 $\mu$l of the reaction stop solution, the absorbance was measured using a microplate meter to analyze the antibody-dependent cellular cytotoxicity inducing ability. Y and X axes represent % maximal cytotoxicity and antibody concentration, respectively.

$$Cytotoxicity\ (\%) = (A-B)/(C-B) \times 100$$

A: Release value upon antibody treatment

B: Spontaneous cell release value

C: Maximum cell release value

The result of the analysis showed that the antibody-dependent cellular cytotoxicity induction of 5R65 and 5R65.7 in eosinophils derived from healthy donors and patients with severe asthma was higher than that of the benralizumab analogue, and 5R65.7 exhibited the strongest induction ability. This can be confirmed by a quantified graph (FIG. 13B) .

[Industrial applicability]

The present invention provides an anti-IL-5R$\alpha$ humanized antibody or antigen-binding fragment thereof, wherein the antibody inhibits the bioactivity of IL-5, suppresses the proliferation of patient-derived eosinophils, and eliminates eosinophils through antibody-dependent cellular cytotoxicity (ADCC).

Therefore, the anti-IL-5R$\alpha$ humanized antibody or antigen-binding fragment thereof according to the present invention can be used for prevention or treatment of allergic diseases, inflammatory diseases, and/or diseases caused by an increase in eosinophils.

Examples of such diseases include, but are not limited to, hypereosinophilic syndrome (HES), hypereosinophilia, asthma including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophiliamyalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, rheumatoid arthritis, and the like.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

[Sequence Listing Free Text]

An electronic file is attached.

**Claims**

1. An antibody or antigen-binding fragment thereof binding to human IL-5Rα recognizing, as an epitope, at least one amino acid residue selected from the group consisting of amino acid residues 221 to 322 corresponding to domain 3 (D3) of a sequence of human IL-5 receptor alpha subunit (IL-5Rα) represented by SEQ ID NO: 19.

2. An antibody or antigen-binding fragment thereof binding to human IL-5 receptor alpha subunit (IL-5Rα) comprises a heavy-chain CDR1 of SEQ ID NO: 3, a heavy-chain CDR2 selected from the group consisting of SEQ ID NOS: 4, 15 to 18, a heavy-chain CDR3 selected from the group consisting of SEQ ID NOS: 5, 27 to 32, and a light-chain CDR1 of SEQ ID NO: 8, a light-chain CDR2 of SEQ ID NO: 9, and a light-chain CDR3 of SEQ ID NO: 10.

3. The antibody or antigen-binding fragment thereof according to claim 1 or 2, wherein the antibody or antigen-binding fragment thereof comprises:

a heavy-chain CDR1 of SEQ ID NO: 3, a heavy-chain CDR2 of SEQ ID NO: 4, and a heavy-chain CDR3 of SEQ ID NO: 5, and a light-chain CDR1 of SEQ ID NO: 8, a light-chain CDR2 of SEQ ID NO: 9, and a light-chain CDR3 SEQ ID NO: 10;
a heavy-chain CDR1 of SEQ ID NO: 3, a heavy-chain CDR2 of SEQ ID NO: 15, a heavy-chain CDR3 of SEQ ID NO: 5 and a light-chain CDR1 of SEQ ID NO: 8, a light-chain CDR2 of SEQ ID NO: 9, and a light-chain CDR3 of SEQ ID NO: 10;
a heavy-chain CDR1 of SEQ ID NO: 3, a heavy-chain CDR2 of SEQ ID NO: 16, a heavy-chain CDR3 of SEQ ID NO: 5 and a light-chain CDR1 of SEQ ID NO: 8, a light-chain CDR2 of SEQ ID NO: 9, and a light-chain CDR3 of SEQ ID NO: 10;
a heavy-chain CDR1 of SEQ ID NO: 3, a heavy-chain CDR2 of SEQ ID NO: 17, a heavy-chain CDR3 of SEQ ID NO: 5 and a light-chain CDR1 of SEQ ID NO: 8, a light-chain CDR2 of SEQ ID NO: 9, and a light-chain CDR3 of SEQ ID NO: 10;
a heavy-chain CDR1 of SEQ ID NO: 3, a heavy-chain CDR2 of SEQ ID NO: 18, a heavy-chain CDR3 of SEQ ID NO: 5 and a light-chain CDR1 of SEQ ID NO: 8, a light-chain CDR2 of SEQ ID NO: 9, and a light-chain CDR3 of SEQ ID NO: 10;
a heavy-chain CDR1 of SEQ ID NO: 3, a heavy-chain CDR2 of SEQ ID NO: 15, a heavy-chain CDR3 of SEQ ID NO: 27 and a light-chain CDR1 of SEQ ID NO: 8, a light-chain CDR2 of SEQ ID NO: 9, and a light-chain CDR3 of SEQ ID NO: 10;
a heavy-chain CDR1 of SEQ ID NO: 3, a heavy-chain CDR2 of SEQ ID NO: 15, a heavy-chain CDR3 of SEQ ID NO: 28 and a light-chain CDR1 of SEQ ID NO: 8, a light-chain CDR2 of SEQ ID NO: 9, and a light-chain CDR3 of SEQ ID NO: 10;
a heavy-chain CDR1 of SEQ ID NO: 3, a heavy-chain CDR2 of SEQ ID NO: 15, a heavy-chain CDR3 of SEQ ID NO: 29 and a light-chain CDR1 of SEQ ID NO: 8, a light-chain CDR2 of SEQ ID NO: 9, and a light-chain CDR3 of SEQ ID NO: 10;
a heavy-chain CDR1 of SEQ ID NO: 3, a heavy-chain CDR2 of SEQ ID NO: 15, a heavy-chain CDR3 of SEQ ID NO: 30 and a light-chain CDR1 of SEQ ID NO: 8, a light-chain CDR2 of SEQ ID NO: 9, and a light-chain CDR3 of SEQ ID NO: 10;
a heavy-chain CDR1 of SEQ ID NO: 3, a heavy-chain CDR2 of SEQ ID NO: 15, a heavy-chain CDR3 of SEQ ID NO: 31 and a light-chain CDR1 of SEQ ID NO: 8, a light-chain CDR2 of SEQ ID NO: 9, and a light-chain CDR3 of SEQ ID NO: 10; or
a heavy-chain CDR1 of SEQ ID NO: 3, a heavy-chain CDR2 of SEQ ID NO: 15, a heavy-chain CDR3 of SEQ ID NO: 32 and a light-chain CDR1 of SEQ ID NO: 8, a light-chain CDR2 of SEQ ID NO: 9, and a light-chain CDR3 of SEQ ID NO: 10.

4. The antibody or antigen-binding fragment thereof according to claim 1 or 2, wherein the antibody or antigen-binding fragment thereof comprises a heavy-chain variable region selected from the group consisting of SEQ ID NOS: 1, 2, 11 to 14, and 21 to 26.

5. The antibody or antigen-binding fragment thereof according to claim 1 or 2, wherein the antibody or antigen-binding fragment thereof comprises a light-chain variable region of SEQ ID NO: 6 or 7.

6. A nucleic acid encoding the antibody or antigen-binding fragment thereof according to any one of claims 1 to 5.

7. An expression vector comprising the nucleic acid according to claim 6.

8. A cell transformed with the expression vector according to claim 7.

9. A method of producing an antibody or antigen-binding fragment thereof binding to human IL-5R$\alpha$ comprising:

(a) culturing the cell according to claim 8 to produce an antibody or antigen-binding fragment thereof; and
(b) recovering the produced antibody or antigen-binding fragment thereof.

10. A conjugate comprising the antibody or antigen-binding fragment thereof according to any one of claims 1 to 5 and a bioactive molecule bound thereto.

11. A bispecific or multispecific antibody comprising the antibody or antigen-binding fragment thereof according to any one of claims 1 to 5.

12. A composition for preventing or treating allergic diseases, inflammatory diseases, and/or diseases caused by an increase in eosinophils comprising the antibody or antigen-binding fragment thereof according to any one of claims 1 to 5.

13. The composition according to claim 12, wherein the allergic diseases, inflammatory diseases, and/or diseases caused by an increase in eosinophils are selected from the group consisting of hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases including atopic dermatitis, allergic diseases including allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophiliamyalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, and rheumatoid arthritis.

14. A composition for diagnosing allergic diseases, inflammatory diseases, and/or diseases caused by an increase in eosinophils comprising the antibody or antigen-binding fragment thereof according to any one of claims 1 to 5.

15. The composition according to claim 14, wherein the allergic diseases, inflammatory diseases, and/or diseases caused by an increase in eosinophils are selected from the group consisting of hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases including atopic dermatitis, allergic diseases including allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophiliamyalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, and rheumatoid arthritis.

【Fig. 1a】

【Fig. 1b】

sIL-5Rα

【Fig. 2a】

【Fig. 2b】

【Fig. 3a】

Benralizumab analogue    m2B7    hu2B7

— 400 nM
— 200 nM
— 100 nM
— 50 nM
— 25 nM

Response Unit (nm)

0.3  0.2  0.1  0.0

Time (sec)

【Fig. 3b】

Antibody concentration
20 nM  100 nM  500 nM

Proliferation of TF-1/IL-5Rα (%)

100

50

0

Vehicle   m2B7   hu2B7   Benralizumab analogue

【Fig. 4】

Antigen binding detection

Biotinylated sIL-5Rα

Expression level detection

| | VH-CDR2 | | | | | | | | | | | | | | | |
|---|---|---|---|---|---|---|---|---|---|---|---|---|---|---|---|
| Kabat No. | 5 0 | 5 1 | 5 2 | 5 2 a | 5 3 | 5 4 | 5 5 | 5 6 | 5 7 | 5 8 | 5 9 | 6 0 | 6 1 | 6 2 | 6 3 | 6 4 | 6 5 |
| Hu2B7 | H | I | Y | P | S | E | S | Y | T | N | Y | N | Q | K | F | K | D |
| Designed codon | | | | X | X | X | X | X | X | | | X | X | | | | |

hu2B7 scFab Library

X = NHB degenerated codon
(F, I, L, M, V, S, P, T, A, Y, H, Q, N, K, D, E)

【Fig. 5a】

【Fig. 5b】

【Fig. 5c】

【Fig. 5d】

【Fig. 6a】

【Fig. 6b】

【Fig. 7a】

PDB(Protein Database) ID: 3QT2

【Fig. 7b】

【Fig. 7c】

【Fig. 8a】

| | VL-CDR3 | | | | | | | | |
|---|---|---|---|---|---|---|---|---|---|
| Kabat No. | 8 9 | 9 0 | 9 1 | 9 2 | 9 3 | 9 4 | 9 5 | 9 6 | 9 7 |
| 5R65 | Q | Q | F | G | R | Y | P | Y | T |
| Designed codon | | | X | X | X | X | X | X | X |

**SEQ ID No: 10**

| | VH-CDR3 | | | | | | | | | | | | |
|---|---|---|---|---|---|---|---|---|---|---|---|---|---|
| Kabat No. | 9 5 | 9 6 | 9 7 | 9 8 | 9 9 | 1 0 0 | 1 0 0 a | 1 0 0 b | 1 0 0 d | 1 0 0 d | 1 0 0 f | 1 0 1 | 1 0 2 |
| 5R65 | D | Y | Y | G | R | S | Y | Y | Y | A | M | D | Y |
| Designed codon | X | X | X | X | X | X | X | X | X | X | | | |

**SEQ ID No: 5**

X : maintaining the original amino acids of 5R65 at a level of ~50% at each residue, using spiked oligonucleotide

【Fig. 8b】

【Fig. 8c】

【Fig. 8d】

5R65 (Abs IC$_{50}$ = 11.78 nM)
5R65.7 (Abs IC$_{50}$ = 0.57 nM)
5R65.10 (Abs IC$_{50}$ = 1.34 nM)
5R65.14 (AbsIC$_{50}$ = 1.13 nM)
5R65.18 (Abs IC$_{50}$ = 1.06 nM)
5R65.39 (Abs IC$_{50}$ = 0.81)
5R65.45 (Abs IC$_{50}$ = 1.26 nM)
Benralizumab analogue (Abs IC$_{50}$ = 1.99 nM)

【Fig. 9a】

# Eosinophils from 7 healthy controls

【Fig. 9b】

# Neutrophils from 7 healthy controls

【Fig. 10a】

Eosinophils from 5 SEA patients

【Fig. 10b】

# Neutrophils from 5 SEA patients

SEA patients

Isotype control

anti-IL-5Rα Ab-PE

SSC

IL-5Rα

Siglec-8

Siglec-8

【Fig. 11a】

【Fig. 11b】

【Fig. 11c】

【Fig. 12a】

【Fig. 12b】

**Patients with SEA**

**Benralizumab analogue / 5R65.7 comparison bar graph**

Proliferation of eosinophils (%) vs Antibody concentration (5 nM, 20 nM, 100 nM)

Legend:
- Isotype control Ab
- 5R65
- 5R65.7
- Benralizumab analogue

【Fig. 13a】

Effector cells (NK cells)

Benralizumab analogue

Release of cytotoxic granules

Target cells (Eosinophils)

FcγRIIIa
IL-5Rα

5R65.7

【Fig. 13b】

Healthy controls

【Fig. 13c】

Patients with SEA

## INTERNATIONAL SEARCH REPORT

| International application No. |
| --- |
| **PCT/KR2021/012345** |

| A. | CLASSIFICATION OF SUBJECT MATTER |
| --- | --- |

C07K 16/28(2006.01)i; A61P 37/08(2006.01)i; A61P 29/00(2006.01)i; G01N 33/68(2006.01)i; A61K 39/00(2006.01)i

According to International Patent Classification (IPC) or to both national classification and IPC

| B. | FIELDS SEARCHED |
| --- | --- |

Minimum documentation searched (classification system followed by classification symbols)

C07K 16/28(2006.01); A61K 39/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: IL-5Rα(interleukin-5 receptor alpha), 도메인 3(domain 3), 항체(antibody), 중쇄 (heavy chain), 경쇄(light chain), 알레르기성 질환(allergic disease), 염증성 질환(inflammatory disease), 호산구(eosinophil)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | US 2003-0096977 A1 (KOIKE, M. et al.) 22 May 2003 (2003-05-22)<br>    See claims 1-3, 5 and 10, and paragraphs [0013]-[0014] and [0017]-[0018]. | 1-5 |
| A | KR 10-2020-0058537 A (JOINT STOCK COMPANY "BIOCAD") 27 May 2020 (2020-05-27)<br>    See entire document. | 1-5 |
| A | US 2005-0226867 A1 (IIDA, S. et al.) 13 October 2005 (2005-10-13)<br>    See entire document. | 1-5 |
| A | NCBI. Genbank accession no. BAE72019.1 (26 July 2016).<br>    See entire document. | 1-5 |
| A | NCBI. Genbank accession no. AAC04540.1 (25 July 2016).<br>    See entire document. | 1-5 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 December 2021** | **24 December 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2021/012345** |

| **Box No. I** | **Nucleotide and/or amino acid sequence(s) (Continuation of item 1.c of the first sheet)** |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

## INTERNATIONAL SEARCH REPORT

| International application No. |
| --- |
| **PCT/KR2021/012345** |

**Box No. II      Observations where certain claims were found unsearchable (Continuation of item 2 of first sheet)**

This international search report has not been established in respect of certain claims under Article 17(2)(a) for the following reasons:

1. ☐   Claims Nos.:
because they relate to subject matter not required to be searched by this Authority, namely:

2. ☑   Claims Nos.: **7-9, 13, 15**
because they relate to parts of the international application that do not comply with the prescribed requirements to such an extent that no meaningful international search can be carried out, specifically:

Claims 7-9, 13 and 15 refer to multiple dependent claims not meeting the requirement of PCT Rule 6.4(a), and thus claims 7-9, 13 and 15 are unclear.

3. ☑   Claims Nos.: **6, 10-12, 14**
because they are dependent claims and are not drafted in accordance with the second and third sentences of Rule 6.4(a).

Form PCT/ISA/210 (continuation of first sheet) (July 2019)

**INTERNATIONAL SEARCH REPORT**
Information on patent family members

| International application No. |
| --- |
| **PCT/KR2021/012345** |

| Patent document cited in search report | | | Publication date (day/month/year) | Patent family member(s) | | | Publication date (day/month/year) |
| --- | --- | --- | --- | --- | --- | --- | --- |
| US | 2003-0096977 | A1 | 22 May 2003 | CN | 1189190 | A | 29 July 1998 |
| | | | | EP | 0811691 | A1 | 10 December 1997 |
| | | | | EP | 0811691 | B1 | 01 December 2004 |
| | | | | JP | 10-010354 | A1 | 24 November 1998 |
| | | | | JP | 3946256 | B2 | 18 July 2007 |
| | | | | KR | 10-0259828 | B1 | 15 June 2000 |
| | | | | KR | 10-1997-0707160 | A | 01 December 1997 |
| | | | | US | 2005-0272918 | A1 | 08 December 2005 |
| | | | | US | 2007-0048304 | A1 | 01 March 2007 |
| | | | | US | 6018032 | A | 25 January 2000 |
| | | | | US | 6538111 | B1 | 25 March 2003 |
| | | | | US | 7179464 | B2 | 20 February 2007 |
| | | | | US | 7238354 | B2 | 03 July 2007 |
| | | | | WO | 97-10354 | A1 | 20 March 1997 |
| KR | 10-2020-0058537 | A | 27 May 2020 | CN | 111788223 | A | 16 October 2020 |
| | | | | EP | 3693388 | A1 | 12 August 2020 |
| | | | | JP | 2021-501568 | A | 21 January 2021 |
| | | | | US | 2020-0291121 | A1 | 17 September 2020 |
| | | | | WO | 2019-070164 | A1 | 11 April 2019 |
| US | 2005-0226867 | A1 | 13 October 2005 | EP | 1688437 | A1 | 09 August 2006 |
| | | | | US | 2008-0095765 | A1 | 24 April 2008 |

Form PCT/ISA/210 (patent family annex) (July 2019)